(19) Europäisches Patentamt
European Patent Office
Office européen des brevets

(11) Numéro de publication : **0 109 317 B1**

# (12) FASCICULE DE BREVET EUROPÉEN

(45) Date de publication du fascicule du brevet : **30.12.86**

(51) Int. Cl.[4] : **C 07 D211/14**, C 07 D211/18, C 07 D211/22, A 61 K 31/445

(21) Numéro de dépôt : **83401939.0**

(22) Date de dépôt : **04.10.83**

(54) **Dérivés de phényl-1 pipéridino-2 propanol, leur préparation, et médicaments qui les contiennent.**

(30) Priorité : **13.10.82 FR 8217187**

(43) Date de publication de la demande : **23.05.84 Bulletin 84/21**

(45) Mention de la délivrance du brevet : **30.12.86 Bulletin 86/52**

(84) Etats contractants désignés : **AT BE CH DE FR GB IT LI LU NL SE**

(56) Documents cités :
**FR-A- 2 070 102**
**FR-A- 2 105 119**
**FR-A- 2 163 358**
**FR-A- 2 208 901**
**FR-A- 2 471 374**
**FR-M- 5 733**
**Le dossier contient des informations techniques présentées postérieurement au dépôt de la demande et ne figurant pas dans le présent fascicule.**

(73) Titulaire : **SYNTHELABO**
**58, rue de la Glacière**
**F-75621 Paris Cedex 13 (FR)**

(72) Inventeur : **Wick, Alexander**
**10, Bd des Plants**
**F-78860 St-Nom-la-Bretèche (FR)**
Inventeur : **Frost, Jonathan**
**7, route de Montjean**
**F-91320 Wissous (FR)**
Inventeur : **Gaudilliere, Bernard**
**28, rue Zilina**
**F-92000 Nanterre (FR)**
Inventeur : **Bertin, Jean**
**55, rue d'Estienne d'Orves**
**F-92140 Clamart (FR)**
Inventeur : **Dupont, Régis**
**1, rue Mademoiselle Villebon s/Yvette**
**F-91120 Palaiseau (FR)**
Inventeur : **Rousseau, Jean**
**17 bis, avenue de Montrouge**
**F-92340 Bourg-la-Reine (FR)**

(74) Mandataire : **Ludwig, Jacques et al**
**SYNTHELABO Service Brevets 58, rue de la Glacière**
**F-75621 Paris Cedex 13 (FR)**

EP 0 109 317 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet des composés répondant à la formule générale (I)

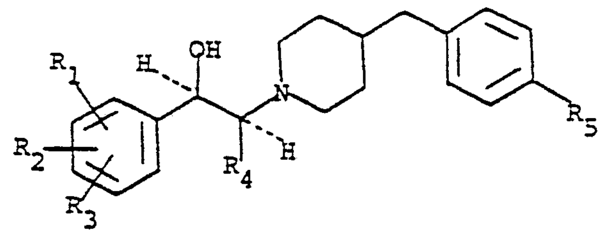

(I)

dans laquelle

$R_1$ représente un atome d'hydrogène, un atome d'halogène, un groupe trifluorométhyle. un groupe alkyle ayant de 1 à 4 atomes de carbone, un groupe hydroxyle, un groupe alcoxyle ayant de 1 à 4 atomes de carbone, un groupe benzyloxyle, un groupe alcanoyloxyle ayant de 1 à 16 atomes de carbone, un groupe benzoyloxyle ou encore, lorsque $R_2$ représente un groupe hydroxyle ou méthoxyle en position 4 et $R_3$ représente un atome d'hydrogène, $R_1$ peut représenter un groupe hydroxyméthyle un groupe carbamoyle, ou un groupe alcoxycarbonyle ayant de 1 à 4 atomes de carbone dans la partie alcoxy,

$R_2$ représente un atome d'hydrogène, un atome d'halogène, un groupe alkyle ayant de 1 à 4 atomes de carbone, un groupe hydroxyle, ou un groupe alcoxyle ayant de 1 à 4 atomes de carbone,

$R_3$ représente un atome d'hydrogène, ou un groupe alkyle ayant de 1 à 4 atomes de carbone,

$R_4$ représente un groupe alkyle ayant 1 à 4 atomes de carbone, les composés étant alors sous forme (±) érythro, ou, lorsque $R_3$ représente un atome d'hydrogène, $R_4$ peut aussi représenter un atome d'hydrogène,

$R_5$ représente un atome d'hydrogène, un atome d'halogène, un groupe alkyle ayant de 1 à 4 atomes de carbone, un groupe alcoxyle ayant de 1 à 4 atomes de carbone, ou un ensemble de trois groupes méthoxyles en positions 3, 4, 5 du radical benzyle,

ainsi que leurs sels d'addition à des acides pharmaceutiquement acceptables, à l'exception des composés de la formule (I) desquels

a) l'un des substituants $R_1$ et $R_2$ est en position 4 et représente un groupe hydroxyle, alcoxyle ou benzyloxyle, l'autre est en position 3 et représente un atome d'hydrogène ou un groupe hydroxyle, alcoxyle ou benzyloxyle, et $R_3$ et $R_5$ représentent chacun un atome d'hydrogène, ou bien

b) $R_1$ ou $R_2$ est en position 4 et représente un atome d'halogène, $R_4$ représente un groupe méthyle, et les autres substituants sont chacun un atome d'hydrogène.

L'invention concerne également les compositions pharmaceutiques contenant les composés de formule (I) ou leurs sels, ainsi qu'un procédé pour les préparer.

Des composés de formule (I) dans laquelle $R_1$ désigne un groupe hydroxyle ou un atome d'halogène en position 4, $R_2$, $R_3$ et $R_5$ désignent chacun un atome d'hydrogène et $R_4$ désigne un groupe méthyle, sont décrits dans le brevet français n° 5733 M et dans la demande de brevet européen n° 63084.

Des composés de formule (I) dans laquelle $R_1$ et $R_2$ désignent un ou deux groupes hydroxyles éventuellement éthérifiés sont décrits dans le brevet français n° 2 163 358.

Parmi les composés de l'invention, sont référés ceux dans la formule (I) desquels

$R_1$ représente H, un halogène, un groupe $CF_3$, un groupe $CH_3$, un groupe OH ou un groupe $OCH_3$,

$R_2$ représente H, un halogène, un groupe $CH_3$, un groupe OH ou un groupe $OCH_3$,

$R_3$ représente H ou un groupe $CH_3$,

$R_4$ représente H ou un groupe $CH_3$, et

$R_5$ représente H, un halogène, un groupe $CH_3$, un groupe $OCH_3$ ou un ensemble de trois groupes $OCH_3$ en position 3, 4 et 5.

Dans cette catégorie de composés, on préfère en particulier ceux dans la formule (I) desquels $R_1$ représente un halogène, $R_2$ et $R_3$ représentent l'hydrogène, $R_4$ représente l'hydrogène ou un groupe méthyle, et $R_5$ représente l'hydrogène ou un halogène.

Une autre catégorie de composés préférés est celle des composés dans la formule desquels

$R_1$ représente $CH_2OH$, $COOCH_3$, $COOC_2H_5$ ou $CONH_2$,

$R_2$ représente OH,

$R_3$ représente H, $R_4$ représente $CH_3$ et $R_5$ représente H.

Les demandes de brevets français 2 208 901 et 2 471 374 décrivent des procédés de préparation d'un composé connu, ifenprodil, ou (hydroxy-4 phényl)-1 (benzyl-4.pipéridino)-2 propanol, dont la structure est proche de celle des composés de formule (I).

Les composés de formule (I) peuvent être préparés selon le schéma de réaction suivant :

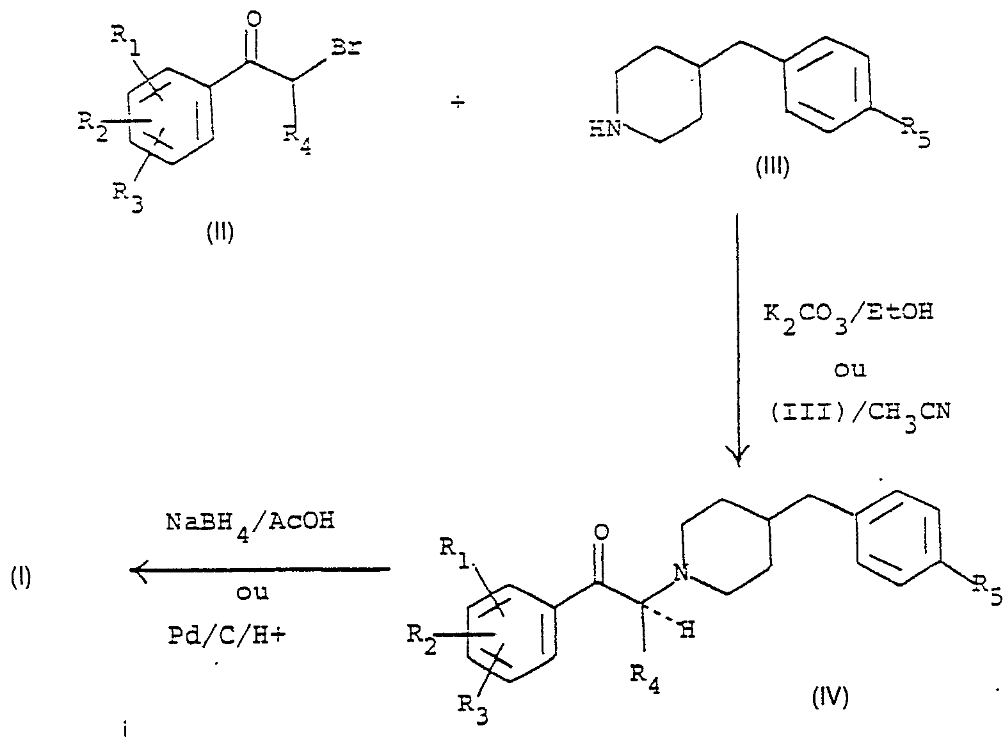


Les acétophénones et propiophénones de formule (II), au moins sous leur forme non bromée en α, sont disponibles dans le commerce ; sinon elles peuvent être préparées à partir de produits du commerce par des procédés connus.

Ainsi par exemple on peut, à partir d'un acide benzoïque convenablement substitué, préparer le chlorure d'acide correspondant et faire réagir celui-ci avec le bromure d'éthylmagnésium ou le diéthylcadmium.

Inversement on peut préparer un diphénylcadmien à partir d'un bromobenzène convenablement substitué et le faire réagir avec le chlorure de propionyle.

On peut également préparer les propiophénones à partir d'un éthylmagnésien et d'un benzonitrile ou d'un benzamide substitué. Enfin, dans le cas où l'un des substituants $R_1$, $R_2$ et $R_3$ est un groupe hydroxyle, on peut estérifier ce dernier par l'acide propionique et soumettre l'ester obtenu à une transposition de Fries.

Les propiophénones non bromées (formule II, avec H au lieu de Br) peuvent être bromées de manière connue, par exemple directement par le brome, en solution dans un solvant approprié tel que le chloroforme, ou dans un mélange de dioxanne et d'éther éthylique, selon une méthode décrite dans la demande de brevet japonais n° 56/43266. On peut également utiliser un agent de bromation sélectif tel que l'hydrotribromure de pyrrolidinone, de formule,

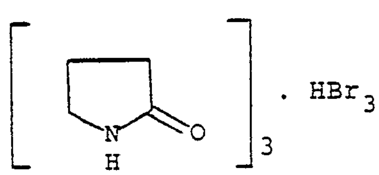

avec du tétrahydrofuranne comme solvant.

La description des procédés ci-dessus s'applique à des propiophénones (formule II, $R_4 = CH_3$. L'homme du métier l'interprétera facilement pour l'appliquer aux acétophénones (formule II, $R_4$ = H).

Les benzylpipéridines de formule (III) sont également disponibles dans le commerce ou, à défaut peuvent être préparées selon des procédés connus.

Ainsi on peut effectuer une réaction de Friedel-Crafts entre le chlorure d'isonicotinoyle et un benzène substitué par $R_5$, réduire la cétone obtenue et hydrogéner l'hétérocycle. On peut également partir de l'acide déjà hydrogéné, à savoir l'acide isonipécotique, l'acyler à l'azote avant de préparer le chlorure, puis faire réagir celui-ci avec un benzène substitué par $R_5$, former l'hydrazone de la cétone obtenue, et finalement le traiter par de la soude en présence d'éthylène glycol pour simultanément décomposer l'hydrazone et déprotéger l'azote hétérocyclique.

La réaction entre les composés de formule II et III se fait en présence d'une base faible minérale telle que le carbonate de sodium ou de potassium, dans un solvant tel qu'un alcool, par exemple l'éthanol, ou

tel qu'une cétone, par exemple la méthyléthylcétone, ou bien encore, dans une proportion de deux moles de benzylpipéridine de formule III pour une mole de propiophénone de formule II, dans l'acétonitrile. C'est alors l'excès de benzylpipéridine qui fixe l'acide bromhydrique libéré par la réaction.

La réaction de réduction de la cétone de formule IV se fait également de manière connue soit par exemple par le borohydrure de sodium ou de potassium en milieu acide, soit par hydrogénation catalytique dans le cas où les symboles $R_1$, $R_2$, $R_3$ et $R_5$ ne désignent pas des atomes d'halogènes.

Les composés de formule I peuvent être eux mêmes transformés en d'autres composés de formule I ; par exemple un groupe hydroxyle peut être transformé en un groupe alcoxyle par une alkylation de manière connue. Des modifications des substituants $R_1$, $R_2$ et $R_3$ peuvent également être effectuées sur la cétone de formule IV, c'est à dire avant la réduction de celle-ci. Ainsi, par exemple, on peut estérifier un groupe hydroxyle, transestérifier un groupe alcoxycarbonyle ou réduire un groupe alcoxycarbonyle en hydroxyméthyle (après avoir, dans ce dernier cas, protégé la fonction cétone) avant de procéder à l'étape finale.

Les exemples suivants illustrent la préparation de quelques composés selon l'invention. Les exemples 1 à 22 concernent des cétones de départ de formule II, les exemples 23 à 25 concernent les benzylpipéridines de formule III et les exemples 26 à 49 concernent les composés finals de formule I.

Les analyses élémentaires et les spectres IR et RMN des composés préparés confirment leurs structures.

Exemples de préparation de cétones de formule II

Exemple 1 Bromo-2 propiophénone.

A 13,4 g (0,1 mole) de propiophénone dans 100 ml de chloroforme anhydre, on ajoute une pincée de chlorure d'aluminium préalablement broyé au mortier puis, en agitant, on y verse au goutte à goutte une solution de 5 ml (0,1 mole) de brome dans 20 ml de chloroforme. Le mélange se décolore instantanément. On refroidit au bain de glace puis on agite une nuit pour chasser l'acide bromhydrique formé. On sépare le catalyseur par filtration, et on chasse le solvant du filtrat. Il reste 22 g d'une huile que l'on utilisera telle quelle dans l'étape suivante.

Exemple 2 Bromo-2 hydroxy-4' propiophénone.

A un mélange de 16 ml de dioxanne et 60 ml d'éther éthylique on ajoute 15 g (0,12 mole) de p-hydroxy-propiophénone. On chauffe la suspension à 30 °C et on ajoute au goutte à goutte 22,4 g (0,14 mole) de brome. On continue d'agiter pendant une heure à température ambiante.

On ajoute alors 40 ml d'eau et 40 ml d'éther, on décante la phase organique, on la lave à l'eau, on la sèche sur sulfate de magnésium et on chasse le solvant. En effectuant une recristallisation avec du toluène on obtient 18 g de fins cristaux lilas qui fondent à 95-97 °C.

Exemple 3 Bromo-2 chloro-4' propiophénone.

A 16,8 g (0,1 mole) de chloro-4' propiophénone en solution dans 100 ml de chloroforme, en présence de chlorure d'aluminium, on ajoute goutte à goutte une solution de 15,9 g (0,1 mole) de brome dans 20 ml de chloroforme, et on agite pendant une nuit. Après filtration et évaporation du filtrat on lave le résidu cristallisé avec de l'éther de pétrole. Sec, il fond à 75 °C.

Exemple 4 Bromo-2 chloro-3' propiophénone.

Dans un ballon tricol de 1 litre, équipé d'un réfrigérant, d'une garde à chlorure de calcium, d'une ampoule isobare, d'un agitateur magnétique, purgé à l'azote, on place 4,86 g (0,2 mole) de tournure de magnésium, 30 ml d'éther éthylique sec et un grain d'iode, puis on y ajoute 21,8 g (0,2 mole) de bromure d'éthyle dans 30 ml d'éther éthylique sec. Ensuite on chauffe le mélange au reflux pendant une heure et on le laisse refroidir. A température ambiante on ajoute alors 16,51 g (0,12 mole) de chloro-3 benzonitrile dans 70 ml d'éther éthylique sec. Un abondant précipité se forme. On agite le mélange une nuit à température ambiante puis on le refroidit au bain de glace et on l'hydrolyse en ajoutant lentement 50 ml d'eau puis environ 100 ml d'acide chlorhydrique 6N jusqu'à un pH acide. On agite l'ensemble pendant une heure et demie puis on l'extrait par de l'acétate d'éthyle. La phase organique est alors lavée deux fois à l'eau, séchée et concentrée à l'évaporateur rotatif. On obtient ainsi 26 g d'une huile orangée que l'on concentre sous vide, d'où il résulte environ 18,2 g de cristaux ocre de chloro-3' propiophénone fondant à environ 40 °C.

On place 18 g (0,1 mole) de ces cristaux dans un erlenmeyer de 250 ml muni d'une ampoule isobare, d'une garde à chlorure de calcium et d'un agitateur magnétique, avec 100 ml de chloroforme et une pincée de chlorure d'aluminium puis, à température ambiante, on ajoute lentement 5,47 ml (0,1 mole) de brome en solution dans 15 ml de chloroforme. On continue d'agiter pendant une heure puis on filtre le mélange sur verre fritté, on concentre le filtrat à l'évaporateur rotatif et l'on obtient une huile orangée que

l'on reprend par 100 ml de chloroforme, lave avec 100 ml de bicarbonate de sodium aqueux à 5 % puis avec 100 ml de thiosulfate de sodium à 5 %. Après séchage sur sulfate de magnésium et concentration à l'évaporateur rotatif il reste 29,8 g d'une huile orangée que l'on peut utiliser telle quelle dans l'étape suivante.

Exemple 5 Bromo-2 chloro-2' propiophénone.

Le mode opératoire est celui de l'exemple 4, sauf que l'on utilise 16,51 g de chloro-2 benzonitrile au lieu de chloro-3 benzonitrile. La chloro-2' propiophénone obtenue après extraction par l'acétate d'éthyle est une huile brune qui distille sous une pression d'environ 2,6 kPa et une température de 120 °C. On en recueille environ 11,0 g que l'on soumet à une bromation telle que décrite à l'exemple 4. Finalement on obtient 26,3 g d'une huile jaune que l'on utilise telle quelle dans l'étape suivante.

Exemple 6 Bromo-2 fluoro-3' propiophénone.

Le mode opératoire est celui de l'exemple 4, sauf que l'on utilise du fluoro-3 benzonitrile au lieu de chloro-3 benzonitrile. La fluoro-3' propiophénone est une huile verdâtre. La bromo-2 fluoro-3' propiophénone est une huile jaune que l'on utilise telle quelle dans l'étape suivante.

Exemple 7 Bromo-2 fluoro-2' propiophénone.

Le mode opératoire est celui de l'exemple 4, sauf que l'on utilise du fluoro-2 benzonitrile au lieu de chloro-3 benzonitrile. La fluoro-2' propiophénone et la bromo-2 fluoro-2' propiophénone sont des huiles incolores.

Exemple 8 Bromo-2 trifluorométhyl-4' propiophénone.

Dans un tricol de 500 ml muni d'un agitateur magnétique et contenant 150 ml de chlorure de thionyle, on introduit par fractions 43,5 g (0,229 mole) d'acide trifluorméthyl-4 benzoïque, puis on chauffe progressivement jusqu'à environ 70 °C. Après 6 heures à cette température on laisse reposer 20 heures puis on chasse le chlorure de thionyle en excès sous vide. Ensuite on distille le résidu huileux sous une pression de 130 à 650 Pa. Une fraction incolore de 42,1 g de chlorure de trifluorométhyl-4 benzoyle passe entre 36 et 40 °C.

A une suspension de 9,6 g de tournures de magnésium, broyées au mortier, dans 120 ml d'éther éthylique sec, on ajoute un cristal d'iode puis, lentement, une solution de 43,6 g (0,4 mole) de bromure d'éthyle dans 80 ml d'éther éthylique sec, tout en refroidissant le mélange pour limiter le reflux du solvant. Ensuite on chauffe à reflux pendant une demi heure, on refroidit le mélange à 5 °C et, par fractions, on ajoute 36,7 g de chlorure de cadmium préalablement séché. La réaction étant très exothermique, on maintient la température à moins de 10 °C. On ajoute encore 100 ml d'éther pour rendre le mélange plus fluide. On chauffe au reflux pendant 40 minutes et on laisse reposer une nuit à température ambiante. On évapore alors l'éther sous vide, on reprend le résidu pâteux par 140 ml de benzène sec, on refroidit entre 5 et 10 °C et on ajoute lentement une solution de 41,7 g (0,2 mole) du chlorure de trifluorométhyl-4 benzoyle, précédemment préparé, dans 60 ml de benzène sec, de façon à maintenir une température inférieure à 10 °C. Après cette addition on laisse revenir à température ambiante, tout en agitant encore pendant une heure pour homogénéiser le mélange, puis on chauffe deux heures au reflux. On refroidit sur bain de glace, jusqu'à 10 °C, puis on verse le mélange dans une solution glacée saturée en chlorure de sodium, tout en agitant. On filtre le précipité et on le lave à l'éther. On sépare les phases du filtrat par décantation, et on extrait la phase aqueuse, laiteuse, à l'éther. On réunit les phases organiques, on les extrait à la soude diluée, on les lave, on les sèche sur sulfate de sodium et on les concentre sous vide. Après distillation sous un vide d'environ 6,5 Pa entre 50 et 56 °C et recristallisation dans l'éther de pétrole on obtient 14,4 g de cristaux blancs de trifluorométhyl-4' propiophénone fondant à 36-37 °C.

Pour la bromation, on dissout 12,25 g de ce composé dans 100 ml de chlorure de méthylène et on additionne goutte à goutte, sous vive agitation, 3,25 ml de brome, dont la décoloration est immédiate. Ensuite on rince avec 20 ml de chlorure de méthylène, on agite à froid pendant une heure et demie et on laisse reposer une nuit. On lave la phase organique à l'eau, jusqu'à un pH neutre, on la sèche et on l'évapore à sec. Le résidu huileux cristallise au congélateur en formant 16,5 g de cristaux incolores.

Exemple 9 Bromo-2 trifluorométhyl-3' propiophénone.

Le mode opératoire est celui de l'exemple 4, sauf que l'on utilise du trifluorométhyl-3 benzonitrile au lieu de chloro-3 benzonitrile.

Exemple 10 Bromo-2 trifluorométhyl-2' propiophénone.

Dans l'appareillage de l'exemple 4 on place 4,86 g (0,2 mole) de tournures de magnésium, 50 ml

d'éther éthylique sec et un grain d'iode puis, à température ambiante, on ajoute lentement 43,88 g (0,195 mole) de bromo-1 trifluorométhyl-2 benzène, de manière à provoquer un léger reflux du solvant. On continue l'agitation pendant une heure puis on ajoute 18,35 g (0,1 mole) de chlorure de cadmium. Un quart d'heure plus tard, la transmétallisation étant réalisée, on concentre le mélange à l'évaporateur rotatif, on lui ajoute du toluène, on concentre à nouveau. On répète ces trois opérations pour éliminer le maximum d'éther.

On ajoute ensuite, à température ambiante, 27,75 g (0,3 mole) de chlorure de propionyle dans 25 ml de toluène puis on chauffe au reflux pendant 2 heures et on laisse refroidir sous azote pendant une nuit. Ensuite on hydrolyse le mélange avec 200 ml d'eau et de l'acide chlorhydrique 6N jusqu'à obtenir un pH de 2 à 3. On extrait le mélange 5 fois avec 200 ml d'acétate d'éthyle, on réunit les phases organiques et on les lave à l'eau jusqu'à obtenir un pH neutre. Après séchage sur sulfate de magnésium et concentration à l'évaporateur rotatif on recueille 34,4 g d'une huile brute que l'on distille sous une pression d'environ 3,3 kPa. A 110 °C il passe une fraction de 14,15 g d'une huile qui, après quelques instants, devient bleuâtre. En effectuant une bromation telle que décrite à l'exemple 4, on obtient une huile verdâtre que l'on utilise telle quelle dans l'étape suivante.

Exemple 11 Bromo-2 méthyl-2' propiophénone.

A une suspension de 10 g de magnésium dans 150 ml d'éther éthylique sec on ajoute 41,8 g de bromure d'éthyle en solution dans 50 ml d'éther éthylique sec. Après 8 heures d'agitation on ajoute par petites portions 35 g de chlorure de cadmium et on laisse reposer une nuit.

Dans un tricol de 2 litres, on place 25 g de chlorure de méthyl-2 benzoyle et 150 ml d'éther éthylique sec, on y introduit peu à peu le mélange ci-dessus, et on maintient l'agitation pendant 30 heures à température ambiante. Ensuite on hydrolyse à l'eau et à l'acide chlorhydrique dilué et on sépare la phase organique que l'on lave à la soude diluée puis à l'eau. Après séchage sur sulfate de sodium et évaporation de l'éther il reste une huile que l'on soumet à une bromation dans 100 ml de chloroforme, en ajoutant 6,5 ml de brome dilué dans 10 ml de chloroforme. Après une heure d'agitation, on filtre et on évapore le filtrat, ce qui laisse 27 g de bromo-2 méthyl-2' propiophénone sous forme d'huile.

Exemple 12 Bromo-2 méthyl-4' propiophénone.

On place 14,8 g (0,1 mole) de méthyl-4' propiophénone dans 100 ml de chloroforme, en présence d'un peu de chlorure d'aluminium finement broyé, et, tout en refroidissant au bain de glace, on ajoute goutte à goutte une solution de 15,9 g (0,1 mole) de brome, soit 5 ml, dans 20 ml de chloroforme. On laisse réagir le mélange pendant une nuit à température ambiante. Ensuite on filtre, on évapore le solvant du filtrat et on lave le résidu cristallisé à l'éther. On recueille 21,5 g de cristaux.

Exemple 13 Bromo-2 méthyl-3' hydroxy-4' propiophénone.

Dans un ballon tricol de 1 litre avec agitation magnétique, réfrigérant à garde de chlorure de calcium et ampoule isobare on place 79,7 g (1,355 mole) d'hydroxy-2 toluène dans 480 ml de toluène, avec 55,3 g, soit 56,3 ml (0,70 mole) de pyridine, puis on y verse lentement 64,75 g, soit 60,8 ml (0,70 mole) de chlorure de propionyle. Il se forme un abondant précipité blanc. Ensuite on chauffe au reflux pendant une heure, on laisse refroidir, puis on ajoute encore 8 ml (0,1 mole) de pyridine et, goutte à goutte, 6,1 ml (0,1 mole) de chlorure de propionyle. On chauffe au reflux pendant une heure, on laisse refroidir, on filtre sur verre fritté pour éliminer le chlorure de pyridinium et on recueille un filtrat orangé qu'on lave successivement avec 200 ml d'acide chlorhydrique 1N, 200 ml de soude 1N, 200 ml d'eau, 200 ml d'acide chlorhydrique 1N, et deux fois 150 ml d'eau. La phase organique ainsi neutralisée est ensuite séchée sur sulfate de sodium et concentrée à l'évaporateur rotatif puis sous vide de la pompe à palettes. Il reste finalement 93,9 g d'une huile brunâtre-orangée.

Une distillation sous une pression de 50 à 90 Pa entre 52 et 58 °C fournit 72,2 g de propionate de méthyl-2 phényle incolore que l'on va soumettre à une transposition de Fries.

Dans un erlenmeyer de 1 litre muni d'une agitation magnétique, d'un réfrigérant, d'une garde à chlorure de calcium et d'une ampoule isobare, sous atmosphère d'azote, contenant 86,4 g (0,648 mole) de chlorure d'aluminium, on verse lentement le propionate ci-dessus, puis on chauffe à 50 °C pendant 3 heures. Il se dégage du chlorure d'hydrogène et le mélange devient une pâte de couleur verte. On laisse reposer 48 heures, puis on hydrolyse le mélange avec 200 ml d'eau et 100 ml d'acide chlorhydrique à 10 %. On extrait le mélange avec du toluène et, en concentrant les phases organiques on obtient une huile brun-jaunâtre que l'on soumet à un entraînement à la vapeur. On acidifie la phase aqueuse ainsi obtenue avec de l'acide chlorhydrique 6N jusqu'à un pH de 2 à 3, on l'extrait une première fois avec 250 ml de toluène, on sature la phase aqueuse restante avec du chlorure de sodium et l'on extrait une deuxième fois avec 250 ml de toluène.

Les deux phases organiques ainsi obtenues sont réunies, séchées et évaporées. Il reste 39,71 g de cristaux brun-clair que l'on recristallise dans un mélange d'éther de pétrole et d'acétate d'éthyle. On recueille finalement 24,8 g de cristaux gris clair de méthyl-3' hydroxy-4' propiophénone, donnant une

seule tache en chromatographie sur couche mince et dont le point de fusion est 86 °C.

Pour la bromation on dissout 8,21 g (0,05 mole) de ces cristaux dans un mélange de 6,6 ml de dioxanne et 25 ml d'éther éthylique, on ajoute goutte à goutte 9,58 g, soit 3,07 ml (0,06 mole) de brome, on agite une nuit à température ambiante, on filtre le précipité, qu'on recueille, et on évapore le filtrat. Les deux solides ainsi obtenus, constituent 14 g de dérivé bromé que l'on utilise tel quel dans l'étape suivante.

Exemple 14 Bromo-2 méthyl-3' méthoxy-4' propiophénone.

On opère comme à l'exemple 13, en faisant précéder l'étape de bromation par une étape de méthylation du groupe hydroxyle.

Pour cela on prépare une solution de 8,2 g (0,95 mole) de méthyl-3' hydroxy-4' propiophénone dans 20 ml d'eau avec 2 g (0,05 mole) de soude en pastilles. On refroidit cette solution à moins de 10 °C et on ajoute goutte à goutte 4,7 ml (0,05 mole) de sulfate de diméthyle. On agite une demi-heure à 100 °C. On laisse refroidir, on extrait l'huile surnageante avec deux fois 50 ml de toluène, on sèche l'extrait et on chasse le solvant. Il reste 9,2 g d'une huile très mobile que l'on soumet à une bromation dans les conditions décrites à l'exemple 13. On obtient ainsi un solide semi cristallisé que l'on utilise tel quel.

Exemple 15 Bromo-2 dichloro-3',5' propiophénone.

On prépare d'abord le chlorure de l'acide dichloro-3,5 benzoïque en portant au reflux 25 g de l'acide libre dans 50 ml de chlorure de thionyle, pendant 4 heures. Puis on évapore l'excès de chlorure de thionyle, on ajoute du toluène et on l'évapore, on recommence l'opération pour éliminer toute trace de chlorure de thionyle.

Par ailleurs, dans 150 ml d'éther éthylique sec contenant 10 g de magnésium on introduit 41,8 g de bromure d'éthyle dilués dans 50 ml d'éther éthylique sec, puis on prépare le dérivé cadmien en ajoutant 35 g de chlorure de cadmium et en laissant réagir une nuit.

On dilue le chlorure d'acide par de l'éther éthylique sec et on y ajoute par petites portions le dérivé cadmien et on laisse agir une nuit. Puis on verse le mélange sur de l'eau glacée, on l'acidifie par de l'acide chlorhydrique 3N, on laisse décanter et on extrait la fraction acqueuse par de l'éther éthylique. On lave les phases organiques à l'eau, on les sèche sur sulfate de sodium, et on chasse le solvant. On obtient 18,6 g d'une huile que l'on dilue avec 70 ml de chloroforme, puis on lui ajoute goutte à goutte 14,34 g soit 4,61 ml de brome dilué dans 25 ml de chloroforme. On laisse réagir pendant 2 heures puis on ajoute de l'eau, on laisse décanter, on lave la phase organique à l'eau, on la sèche et on évapore le chloroforme. On recueille 20 g d'une huile brune que l'on utilise telle quelle.

Exemple 16 Bromo-2 dichloro-2',4' propiophénone.

Dans un tricol de 500 ml contenant 200 ml d'ammoniaque concentrée (25 %), refroidi au bain de glace, on introduit sous azote, goutte à goutte, 43 g de chlorure de dichloro-2,4 benzoyle. Il se forme un précipité abondant qu'on essore après une heure et qu'on lave à l'eau. Après séchage en étuve sous vide, on recueille 42 g d'amide fondant à 193 °C. Par ailleurs on prépare dans 300 ml d'éther éthylique sec de l'iodure d'éthylmagnésium à partir de 19,8 g de magnésium et 88,9 g d'iodure d'éthyle. On lui ajoute alors, par portions 41,1 g de l'amide précédemment préparé. La réaction est exothermique. Après la fin de l'addition on chauffe au reflux la suspension pendant 4 heures, on laisse reposer une nuit, on reprend le chauffage à reflux pendant 8 heures et on laisse reposer une nuit. On verse alors le mélange sur 1,5 litre d'eau glacée, on acidifie avec de l'acide chlorhydrique concentré, ce qui solubilise la phase aqueuse, puis on laisse décanter. On extrait la phase aqueuse à l'éther, on lave les extraits à l'eau, on les sèche et on chasse l'éther. Il reste une suspension huileuse dont on élimine l'amide qui n'a pas réagi par filtration. On dilue le filtrat sirupeux par du chloroforme, on le traite par du charbon décolorant et du sulfate de sodium, et on le filtre. Après évaporation il reste 39,8 g d'une huile brune que l'on soumet à la bromation. Pour cela on la dilue dans 200 ml de chloroforme, on ajoute un cristal de chlorure d'aluminium et on y verse goutte à goutte 10 ml de brome dilués dans 40 ml de chloroforme. Tout en maintenant l'agitation on ajoute du thiosulfate de sodium aqueux, puis on laisse décanter, on lave la phase organique à l'eau, on la sèche et on chasse le solvant. Il reste 51 g d'une huile marron brute que l'on utilise telle quelle.

Exemple 17 Bromo-2 diméthyl-3',5' propiophénone.

On prépare d'une part le chlorure de l'acide diméthyl-3,5 benzoïque et d'autre part le diéthyl cadmium, dans les conditions de l'exemple 15. Puis dans un tricol de 2 l contenant 26 g du chlorure d'acide et 200 ml d'éther éthylique sec on ajoute l'organocadmien et on laisse réagir pendant 48 heures. On verse alors le mélange sur de la glace pilée, on l'acidifie avec de l'acide chlorhydrique 3N, on sépare la phase éthérée, on la lave avec de la soude 6N puis avec de l'eau, on la sèche et on l'évapore. Il reste 26 g d'une huile dont on dilue la moitié dans 100 ml de chloroforme et on lui ajoute 4,2 ml de brome dilués dans 50 ml de chloroforme. Après cette addition on agite pendant 2 heures à température ambiante, on

évapore le solvant, on reprend le résidu avec 100 ml de chloroforme, que l'on évapore à nouveau. Il reste 21 g d'une huile qu'on utilise telle quelle.

Exemple 18 Bromo-2 hydroxy-4' fluoro-3' propiophénone.

Dans un ballon muni d'une ampoule isobare, d'un réfrigérant avec une garde à chlorure de calcium et d'une agitation magnétique, contenant 400 ml de toluène, 60 g (0,54 mole) de fluoro-2 phénol et 55,3 g (0,70 mole) de pyridine, on introduit lentement, à température ambiante, 64,75 g (0,70 mole) de chlorure de propionyle. Après une heure d'agitation, on sépare le précipité blanc par filtration, et on lave le filtrat avec de l'eau, jusqu'à obtenir un pH de 7. Après séchage et évaporation du solvant on recueille 90 g d'ester sous forme d'huile.

Dans un erlenmeyer de 1 litre avec ampoule isobare, agitateur, réfgrigérant à garde de chlorure de calcium on place 133,3 g (1 mole) de chlorure d'aluminium et on y verse lentement 82 g de l'huile ci-dessus.

Puis on porte le mélange à 165 °C pendant 2 heures. Ensuite on l'hydrolyse avec de l'eau, puis avec de l'acide chlorhydrique 6N, on l'extrait avec de l'acétate d'éthyle, on le sèche, on le filtre et on chasse le solvant. Il reste 70 g de cristaux noirs, huileux que l'on dissout avec du cyclohexane chaud. Le refroidissement de la solution provoque un précipité jaune clair. En recommençant la recristallisation on recueille finalement 30 g de cristaux blancs qui fondent à 101-102 °C. La bromation s'effectue dans les mêmes conditions que celles de l'exemple 13.

Exemple 19 Bromo-2 hydroxy-4' méthoxycarbonyl-3' propiophénone.

Dans un tricol de 2 litres avec agitateur mécanique, réfrigérant de thermomètre, on place 600 ml de dichloro-1,2 éthane, 76 g, soit 65 ml (0,5 mole) de salicylate de méthyle et 50,9 g soit 48 ml (0,5 mole) de chlorure de propionyle. On refroidit à 0 °C et on ajoute par petites quantités 133 g (1 mole) de chlorure d'aluminium broyé, de telle façon que la température reste inférieure à 15 °C. Ensuite on agite encore pendant 4 heures à température ambiante et on laisse reposer une nuit. Puis on jette le mélange sur 2 litres d'eau de glace, on sépare la phase organique, on la lave à l'eau, au bicarbonate de sodium, et de nouveau à l'eau. Après séchage et évaporation on obtient une huile qui cristallise dans l'hexane. On recueille 93 g de cristaux fondant à 62-64 °C.

Pour la bromation on place dans un erlenmeyer de 250 ml équipé d'un thermomètre, d'un réfrigérant et d'une ampoule à brome, 70 ml d'éther éthylique, 17 ml de dioxanne et 26 g de la propiophénone précédemment obtenue. On ajoute alors goutte à goutte 7,4 ml de brome, en refroidissant le mélange au bain de glace. On maintient l'agitation pendant 4 heures, puis on jette le mélange sur de l'eau glacée, on décante, on lave la phase organique par trois fois 250 ml d'eau, on la sèche sur sulfate de magnésium et on l'évapore à sec. On isole ainsi 30 g d'un produit qu'on recristallise dans un mélange 80/20 d'éther isopropylique/toluène. On recueille finalement 22 g de cristaux qui fondent à 102 °C.

Exemple 20 Bromo-2 hydroxy-4' diméthyl-3',5' propiophénone.

A 500 ml de toluène contenant 61 g (0,5 mole) de diméthyl-2,6 phénol on ajoute 92 g (1 mole) de chlorure de propionyle puis, goutte à goutte, tout en agitant, 79 g de pyridine.

On chauffe ensuite au reflux pendant 1 heure, on refroidit, on verse le mélange dans 1 litre d'eau à 3 % d'acide chlorhydrique, on agite, on laisse décanter la phase organique, on la sépare, on la lave à l'eau, on la sèche sur sulfate de sodium et l'évapore. On obtient 100 g d'une huile très mobile que l'on utilise telle quelle pour la transposition. Pour cela on la dissout dans 300 ml de chlorobenzène et on verse cette solution lentement sur 100 g de chlorure d'aluminium. On chauffe ensuite le mélange à 80 °C, en agitant pendant 5 heures, on laisse refroidir et on le verse lentement dans un mélange de 850 ml d'eau glacée et 100 ml d'acide chlorhydrique concentré. On agite pendant une demi-heure sur le bain de glace et on filtre le précipité. On le lave, on l'essore et on le sèche. On en recueille 41 g.

On en dissout 17,8 g dans un mélange de 13,2 ml de dioxanne et 50 ml d'éther éthylique sec, à 30 °C, puis on ajoute goutte à goutte 6,1 ml de brome, on agite une nuit à température ambiante et on évapore les solvants. On utilise le résidu tel quel dans l'étape suivante.

Exemple 21 Bromo-2 méthoxy-4' diméthyl-3',5' propiophénone.

On opère comme à l'exemple 20, en faisant précéder l'étape de bromation par une étape de méthylation.

Pour cela on place 4,45 g d'hydroxy-4' diméthyl-3',5' propiophénone dans un ballon de 50 ml avec 10 ml d'eau et 1 g de soude. On refroidit le mélange en dessous de 10 °C et on lui ajoute goutte à goutte 2,35 ml de sulfate de diméthyle.

On chauffe ensuite au bain d'huile à 100 °C pendant une demi-heure et on laisse reposer une nuit à température ambiante. On extrait avec 20 ml de toluène, on laisse décanter, on sèche la phase organique, on l'évapore, et on recueille 4,2 g d'une huile mobile que l'on dilue dans un mélange de 4 ml de dioxanne

et 12 ml d'éther éthylique, on y ajoute goutte à goutte 1,13 ml de brome, on agite 1 heure à température ambiante, on laisse reposer une nuit et on chasse les solvants. Il reste 5,9 g d'une huile qu'on utilisera telle quelle.

Exemple 22 bromo-2 éthyl-4' propiophénone.

Tout en agitant on chauffe pendant 2 à 3 heures, à 80 °C, jusqu'à dissolution complète, un mélange de 100 g d'acide éthyl-4 benzoïque et de 250 ml, soit 153 g de chlorure de thionyle. Ensuite on laisse refroidir, on évapore l'excès de chlorure de thionyle sous vide, et on distille le résidu liquide sous environ 13 Pa à 90 °C. On recueille 95,2 g du chlorure d'acide sous forme d'un liquide incolore.

On prépare ensuite du diéthylcadmium comme décrit à l'exemple 8, à partir de 63 g de bromure d'éthyle, 13,8 g de magnésium et 53 g de chlorure de cadmium. On le met en suspension dans 400 ml de benzène puis, tout en refroidissant à moins de 10 °C, on ajoute goutte à goutte une solution de 48,7 g du chlorure d'éthyl-4 benzoyle précédemment préparé dilué dans 100 ml de benzène. Après l'addition on agite 1 heure à température ambiante puis 2 heures à la température du reflux. Ensuite on refroidit le mélange à 10 °C et on le verse sur un mélange de glace et de sel tout en agitant, on filtre, on sépare la phase organique du filtrat, et on l'extrait deux fois avec de la soude diluée, on la lave à l'eau, on la sèche sur sulfate de magnésium et on l'évapore. Il reste 48,4 g d'un résidu huileux que l'on distille sous vide poussé (13 Pa). Entre 65 et 80 °C il passe une fraction de 35,6 g d'éthyl-4' propiophénone pratiquement pure.

Pour la bromation on en dissout 34,8 g dans 1 000 ml de tétrahydrofuranne, on additionne 17,5 ml de pyrrolidinone puis 114 g d'hydrotribromure de pyrrolidinone. Tout en agitant on chauffe le mélange au reflux ; il se décolore progressivement et le bromhydrate de pyrrolidinone précipite. Après 1 heure la réaction est complète, on essore le bromhydrate, on le rince à l'acétate d'éthyle et on évapore les solvants du filtrat. On reprend le résidu orangé huileux partiellement cristallisé avec un mélange d'eau et de chlorure de méthylène. On sépare la phase organique, on la lave deux fois à l'eau, on la sèche et on l'évapore sous vide. On dissout le résidu huileux verdâtre dans du toluène, on ajoute du pentane, on sépare par filtration sur papier une petite fraction gommeuse verdâtre, on évapore le filtrat. Il reste un résidu huileux que l'on utilise tel quel.

Exemples de préparation de benzylpipéridines de formule III.

Exemple 23 (Méthyl-4 benzyl)-4 pipéridine.

Dans un ballon de 1 litre avec réfrigérant et garde à chlorure de calcium on place 123 g (1 mole) d'acide isonicotinique et on ajoute, en une seule fois, et tout en refroidissant, au bain de galce, 250 ml de chlorure de thionyle. La réaction exothermique, dure une dizaine de minutes. On place alors le mélange sur bain d'huile et on chauffe au reflux pendant une heure et demie. On chasse l'excès de chlorure de thionyle et on reprend le résidu cristallisé par 100 ml d'éther éthylique sec. On agite et on essore le précipité blanc. On le lave encore avec 50 ml d'éther et on le sèche. Il représente 171 g de chlorure d'isonicotinoyle sous forme de chlorhydrate. On en place 92 g (0,516 mole) dans un tricol de 2 litres avec thermomètre, agitateur, réfrigérant, garde à chlorure de calcium, et on ajoute 300 ml de toluène anhydre. On refroidit à 10 °C et on additionne, par petites fractions, 262 g (1,96 mole) de chlorure d'aluminium broyé et on laisse agiter 2 heures à température ambiante. On transvase ensuite la solution rouge sombre dans une ampoule à brome et on la verse goutte à goutte dans 2,5 litres d'eau glacée, refroidis par un bain de glace. Puis on ajoute 800 ml d'acétate d'éthyle et, tout en refroidissant, 1 litre de lessive de soude (d=1,33). On obtient ainsi une solution dont on décante la phase organique. On extrait la phase aqueuse encore trois fois avec 500 ml d'acétate d'éthyle, on réunit les phases organiques, on les lave à l'eau, on les sèche et on évapore le solvant sous vide au bain marie. On triture le résidu avec 100 ml d'éthanol, en refroidissant dans un bain de glace. On l'isole par filtration et on le sèche. Il représente 80 g de (méthyl-4 benzoyl)-4 pyridine. On ajoute alors 42,6 ml d'hydrate d'hydrazine et 18,4 ml d'eau, puis 420 ml d'éthylèneglycol, puis 103,5 g de soude en pastilles. On chauffe au reflux pendant 2 heures, on refroidit et on ajoute 1 litre d'eau. On extrait l'huile surnageante avec quatre fois 250 ml d'éther éthylique. On lave la phase éthérée à l'eau, on la sèche et on l'évapore. On obtient 72 g de (méthyl-4 benzyl)-4 pyridine sous forme d'une huile qui cristallise. On met celle-ci en solution dans 200 ml de méthanol, on y ajoute 2 g de rhodium sur charbon à 5 % et on effectue une hydrogénation sous 5 MPa à environ 70 °C pendant 8 heures. Ensuite on sépare le catalyseur par filtration et on évapore le solvant. Il reste 72 g de (méthyl-4 benzyl)-4 pipéridine sous forme d'huile.

Exemple 24 (Méthoxy-4 benzyl)-4 pipéridine.

On opère comme à l'exemple 23, en remplaçant le toluène par de l'anisole.

Exemple 25 (Fluoro-4 benzyl)-4 pipéridine.

Dans un réacteur de 4 litres muni d'un agitateur mécanique, d'un réfrigérant et d'un thermomètre on chauffe au reflux un mélange de 300 g (2,32 moles) d'acides isonipécotique et 1 200 ml d'anhydride acétique, pendant 2 heures, et on laisse reposer une nuit. Au mélange qui commence à cristalliser on ajoute 1 litre d'éther éthylique, on filtre, et on lave le gâteau avec quatre fois 500 ml d'éther, on l'essore et on le recristallise dans un mélange 40/60 d'éther isopropylique/alcool isopropylique. On isole 153 g dans un premier jet et 141 g dans un second jet. L'acide N-acétyl-isonipécotique fond à 148 °C.

On en introduit 141 g par petites fractions dans un ballon de 4 litres muni d'un agitateur et contenant 860 ml de chlorure de thionyle. Le chlorure d'acide précipite. On agite encore 2 heures puis on ajoute 2 l d'éther de pétrole. On sépare le précipité par filtration, on l'essore, on le lave trois fois avec 500 ml d'éther de pétrole et on le sèche sous vide pà 40 °C. On en isole 140 g qui fondent à enviorn 122 °C. A 350 ml de fluorobenzène placés dans un tricol de 2 litres équipé d'un agitateur et d'un réfrigérant, on ajoute 198 g de chlorure d'aluminium par petites quantités. A cette suspension on ajoute alors par petites quantités 150 g du chlorure d'acide précédemment préparé, en maintenant la température à environ 20 °C grâce à un bain de glace. Ensuite on chauffe au reflux pendant une heure, puis on jette le mélange sur 2 kg de glace et 1 litre d'eau. On extrait le produit formé avec du chlorure de méthylène. On sépare la phase organique, on la lave à l'eau, on la sèche et on l'évapore. On recueille 170 g de N-acétyl-(fluoro-4 benzoyl)-4 pipéridine sous forme d'huile. On en ajoute 12,3 g à 5,375 ml d'hydrate d'hydrazine et 2,3 ml d'eau, on ajoute 150 ml d'éthanol, et on chauffe le tout au reflux pendant 8 heures, on évapore à sec, on reprpend le résidu huileux par de l'éther éthylique. Après 30 minutes d'agitation l'hydrazone cristallise, on l'isole par filtration et on l'essore. On en recueille 10 g qui, après recristallisation dans un mélange d'éther isopropylique/éthanol, fondent à 145-148 °C.

On chauffe pendant 2 heures à 160 °C un mélange de 26 g de cette hydrazone, 27 g de soude et 200 ml d'éthylèneglycol. Puis on jette le mélange sur 500 ml d'eau glacée et on l'extrait par du chloroforme. On sèche la phase organique, on l'évapore, on distille l'huile résiduelle et on recueille une fraction de 10,5 g qui distille entre 104 et 110 °C sous 50 Pa. On en prepare le chlorhydrate en disolvant cette huile orangée dans de l'acétone et en lui ajoutant de l'éther chlorhydrique. Après recristallisation dans un mélange 98/2 acétone/éthanol le chlorhydrate fond à 158-160 °C.

Exemples de préparation de composés selon l'invention.

Exemple 26 [(Méthyl-4 benzyl)-4 pipéridino]-2 (hydroxy-4 phényl)-1 propanol et son chlorhydrate.

1. [(Méthyl-4 benzyl)-4 pipéridino]-2 hydroxy-4' propiophénone.

On place 7,57 g (0,04 mole) de (méthyl-4 benzyl)-4 pipéridine dans 35 ml d'éthanol et on leur ajoute 9,15 g (0,04 mole) de bromo-2 hydroxy-4' propiophénone, puis 4,24 g (0,04 mole) de carbonate de sodium, et on chauffe au reflux pendant 2 heures. Ensuite on refroidit le mélange par un bain de glace et on lui ajoute 200 ml d'eau glacée, puis 100 ml de toluène. On agite, laisse décanter et sépare la phase organique. On extrait la phase aqueuse trois fois avec 100 ml de toluène. On réunit les phases organiques, les lave, les sèche puis les évapore. On obtient ainsi 13 g d'une huile pâteuse que l'on purifie par une chromatographie sur silice en éluant avec de l'acétone. On recueille 10 g d'une huile pâle que l'on utilise telle quelle pour la réduction.

2. [(Méthyl-4 benzyl)-4 pipéridino]-2 (hydroxy-4 phényl)-1 propanol et son chlorhydrate, sous forme (±) érythro.

Dans un flacon à réaction de Parr on dissout 12 g (0,035 mole) de la cétone précédemment obtenue dans 120 ml de méthanol. On y ajoute 20 ml d'acide acétique et on purge le récipient avec de l'azote. Puis on y introduit 1,2 g de charbon palladié à 10 % et on hydrogène à 50 °C pendant 6 heures sous une pression d'environ 0,35 MPa. Ensuite on empoisonne le catalyseur avec 50 ml de chloroforme, on le sépare par filtration et on évapore le filtrat sous vide au bain marie. On reprend le résidu cristallisé avec de l'eau, de l'acétate d'éthyle et de l'ammoniaque. On sépare la phase organique, la lave, la sèche et l'évapore. On reprend le résidu avec 50 ml d'éther et on l'agite 30 minutes sur bain de glace. On filtre le précipité blanc et on le chromatographie sur 50 g de silice en éluant avec de l'acétone. On recueille finalement 3,5 g de cristaux blancs du produit sous forme de base. Pour en préparer le chlorhydrate, on les dissout dans 50 ml d'éthanol absolu, on ajoute 20 ml d'éthanol saturé d'acide chlorhydrique gazeux, on agite pendant 15 minutes, on chasse l'éthanol à l'évaporateur rotatif, on reprend le résidu par 60 ml d'éther anhydre, on agite pendant 15 minutes, on filtre le précipité blanc, on le lave à l'éther, on le dissout dans 200 ml de n-propanol, que l'on concentre à 100 ml, et on laisse le mélange une nuit au réfrigérateur. Ensuite on filtre les cristaux blancs obtenus, on les lave avec de l'éther anhydre et on les sèche. Ils fondent à 223-225 °C.

Exemple 27 (Benzyl-4 pipéridino)-2 phényl-1 propanol et son chlorhydrate.

1. (Benzyl-4 pipéridino)-2 propiophénone.

A une solution de 21,3 g (0,1 mole) de bromo-2 propiophénone, dans 100 ml d'éthanol, on ajoute 17,5 g (0,1 mole) de benzyl-4 pipéridine, puis 12 g de carbonate de sodium et on chauffe au reflux durant 2 heures. Ensuite on filtre et on chasse le solvant. Il reste une huile que l'on utilise telle quelle pour la réduction.

2. (Benzyl-4 pipéridino)-2 phénpyl-1 propanol et son chlorhydrate, sous forme (±) érythro.

On dissout 15 g (0,048 mole) de l'huile précédemment obtenue dans 150 ml d'éthanol, on ajoute 75 ml d'acide acétique puis peu à peu, et en refroidissant le mélange, 10 g de borohydrure de potassium, en agitant pendant une heure à température ambiante. On ajoute alors 200 ml de glace, puis 200 ml de chlorure de méthylène, et on basifie le mélange avec de l'ammoniaque. La phase organique lavée, séchée et évaporée livre un résidu cristallisé qu'on lave à l'éthanol puis à l'éther de pétrole. On prépare le chlorhydrate en lui ajoutant 80 ml d'éthanol puis 20 ml d'éthanol saturé par de l'acide chlorhydrique gazeux. On chauffe le mélange au reflux pendant 5 minutes, on le filtre à chaud et le laisse refroidir. On essore le résidu avec un peu d'éthanol et on le sèche. Il se sublime vers 250 °C et se décompose à 259-262 °C.

Exemple 28 (Benzyl-4 pipéridino)-2 (méthyl-4 phényl)-1 propanol et son chlorhydrate.

1. (Benzyl-4 pipéridino)-2 méthyl-4' propiophénone.

On place 20,5 g (0,09 mole) de bromo-2 méthyl-4' propiophénone dans 100 ml d'éthanol, on ajoute 16 g (0,09 mole) de benzylpipéridine, puis 12 g de carbonate de sodium, puis on chauffe au reflux pendant 2 heures. Une chromatographie en couche mince montre que la réaction est complète. On filtre le mélange et on chasse le solvant. On obtient une huile que l'on utilise telle quelle pour la réduction.

2. (Benzyl-4 pipéridino)-2 (méthyl-4 phényl)-1 propanol et son chlorhydrate, sous forme (±) érythro.

On place 15 g (0,046 mole) de (benzyl-4 pipéridino)-2 méthyl-4' propiophénone dans 150 ml d'éthanol et on ajoute 75 ml d'acide acétique. On plonge le récipient dans un bain de glace et, tout en agitant, on ajoute lentement 10 g de borohydrure de potassium. On continue ensuite l'agitation pendant 1 heure à température ambiante. On ajoute alors 400 ml de glace, puis 200 ml de chlorure de méthylène, et on rend le mélange basique avec de l'ammoniaque concentrée. On lave la solution organique, on la sèche et on l'évapore. Il se forme un produit cristallisé qui, lavé à l'éthanol puis à l'éther de pétrole, puis séché, fond à 118 °C (Koffler). Avec de l'éthanol chlorhydrique on prépare le chlorhydrate qui se sublime vers 250 °C, et se décompose à 251-254 °C.

Exemple 29 [(Méthoxy-4 benzyl)-4 pipéridino]-2 (chloro-4 phényl)-1 propanol et son chlorydrate.

1. [(Méthoxy-4 benzyl)-4 pipéridino]-2 chloro-4' propiophénone.

A une solution de 5 g (0,02 mole) de bromo-2 chloro-4' propiophénone et 4,15 g (0,02 mole) de (méthoxy-4 benzyl)-4 pipéridine dans 60 ml d'éthanol sec on ajoute 2,80 g (0,02 mole) de carbonate de potassium et on agite en chauffant au reflux pendant 5 heures puis on laisse reposer 48 heures. On laisse refroidir, on filtre, en lavant le gâteau de filtration à l'alcool, et on évapore le filtrat. Il reste un résidu huileux orangé que l'on reprend par un mélange d'eau et d'éther. On sépare la phase organique et on extrait la phase aqueuse une deuxième fois à l'éther. On réunit les phases organiques, on les sèche et on les évapore, ce qui fournit 7,45 g d'un résidu huileux jaune-orangé qui cristallise partiellement à température ambiante et qu'on utilise tel quel pour la réduction.

2. [(Méthoxy-4 benzyl)-4 pipéridino]-2 (chloro-4 phényl)-1 propanol et son chlorhydrate, sous forme (±) érythro.

On procède à la réduction comme à l'exemple 26.2, avec les 7,45 g de la cétone précédemment préparée, 2,5 g de borohydrure de potassium, 50 ml d'éthanol et 20 ml d'acide acétique. On agite d'abord une demi heure sur bain de glace, puis 3 heures à température ambiante, on additionne de l'eau puis de l'ammoniaque concentrée et on extrait deux fois au chlorure de méthylène. On lave, sèche et évapore les phases organiques réunies, et on triture le résidu dans de l'éther, obtenant ainsi 5,5 g de cristaux. On en dissout 5,4 g dans 100 ml de méthanol et on ajoute 145 ml de solution chlorhydrique 0,1N d'alcool isopropylique, on évapore la solution et on recristallise le résidu dans 200 ml d'alcool isopropylique. On récupère 4,25 g de cristaux fondant à 201-202 °C.

Exemple 30 [(Fluoro-4 benzyl)-4 pipéridino]-2 (chloro-4 phényl)-1 propanol et son chlorhydrate.

1. [(Fluoro-4 benzyl)-4 pipéridino]-2 chloro-4' propiophénone.

On alcalinise avec de la soude concentrée une solution de 4,95 g de chlorhydrate de (fluoro-4 benzyl)-4 pipéridine dans le minimum d'eau, et on extrait le mélange 3 fois avec du chlorure de méthylène et de l'acétate d'éthyle. Après lavage, séchage et évaporation des phases organiques on est en présence de 4,3 g de la base, sous forme d'huile incolore. On les dissout dans 60 ml d'éthanol, on ajoute 5 g de bromo-2 chloro-4' propiophénone et 2,8 g de carbonate de potassium et on chauffe au reflux, tout en agitant, pendant 5 heures. On laisse reposer une nuit à température ambiante, on sépare le sel minéral, on évapore la phase alcoolique à sec, on reprend le résidu huileux par de l'éther, on sèche la phase organique obtenue, on la filtre et on lui ajoute de l'éther chlorhydrique. Le chlorhydrate précipite sous forme de gomme difficile à essorer. On l'extrait à l'eau, sépare la phase aqueuse acide, et on ajoute à nouveau de l'éther chlorhydrique à la phase éthérée. On recommence ainsi trois fois, puis on alcalinise les phases aqueuses réunies avec de la soude, on extrait la base à l'éther puis à l'acétate d'éthyle. Les phases organiques réunies, lavées, séchées puis évaporées fournissent 5,8 g d'un résidu huileux jaunâtre, qu'on réduira tel quel.

2. [(Fluoro-4 benzyl)-4 pipéridino]-2 (chloro-4 phényl)-1 propanol et son chlorhydrate sous forme (±) érythro.

On procède comme à l'exemple 26.2, avec 5,8 g de la cétone précédemment préparée, 1,7 g de borohydrure de potassium, 160 ml d'éthanol et 50 ml d'acide acétique. On obtient 4,55 g du composé final sous forme de base, après trituration dans l'éther. Après recristallisation dans l'alcool isopropylique et séchage, le chlorhydrate fond à 211-213 °C.

Exemple 31 (Benzyl-4 pipéridino)-2 (chloro-3 phényl)-1 propanol et son chlorhydrate.

1. (Benzyl-4 pipéridino)-2 chloro-3' propiophénone.

Dans un ballon tricol de 500 ml muni d'une ampoule isobare, d'une garde à chlorure de calcium et d'une agitation magnétique on place 26,48 g (0,107 mole) de bromo-2 chloro-3' propiophénone en solution dans 70 ml d'acétonitrile puis, à température ambiante, sans agiter, et en l'espace de 20 minutes environ, on ajoute 37,5 g (0,214 mole) de benzyl-4 pipéridine. Le mélange s'échauffe ; on laisse revenir à température ambiante et, après 3 heures, on cristallise le bromhydrate de benzyl-4 pipéridine en refroidissant le mélange sur glace et en le triturant. On sépare les cristaux par filtration, on les lave avec deux fois 10 ml d'acétonitrile, on dilue le filtrat avec 200 ml d'éther éthylique. Il précipite encore du bromhydrate de benzyl-4 pipéridine que l'on sépare par filtration. On ajoute 100 ml d'acide chlorhydrique 3 N au filtrat et on agite. Il se forme trois phases. On recueille les deux phases inférieures, on reprend la phase supérieure par quelques ml d'eau, et on réunit la phase aqueuse obtenue avec les deux phases précédemment séparées. Il se produit une précipitation. On refroidit le mélange sur glace, on l'agite, on le filtre sur verre fritté et on le sèche en étuve sous vide. Le filtrat dépose un deuxième précipité dont une chromatographie sur couche mince révèle qu'il s'agit du même produit que le premier jet. On récupère ainsi un total de 34,06 g de cristaux de chlorhydrate fondant entre 189 et 198 °C que l'on utilise tels quels pour la réduction.

2. (Benzyl-4 pipéridino)-2 (chloro-3 phényl)-1 propanol et son chlorhydrate sous forme (±) érythro.

A 34,06 g (0,09 mole) du chlorhydrate de la cétone précédemment obtenue on ajoute 300 ml d'éthanol absolu et 150 ml d'acide acétique. On refroidit sur glace et on ajoute peu à peu 20,5 g (0,38 mole) de borohydrure de potassium en maintenant la température inférieure à 20 °C. On agite pendant 2 heures à température ambiante, on refroidit sur bain de glace et on ajoute 350 ml d'eau, puis 220 ml d'ammoniaque à 27 % et 400 ml d'acétate d'éthyle. On sépare les cristaux formés par filtration, et on lave la phase organique à l'eau. On reprend les cristaux par 100 ml d'ammoniaque à 27 % et on ajoute 150 ml de chloroforme. La dissolution des cristaux est complète. On sépare la phase organique, on la lave à l'eau et on l'ajoute à la phase d'acétate d'éthyle issue de la filtration. On sèche le mélange des deux phases organiques et on l'évapore sous vide. On recueille 36,1 g de cristaux blancs. Après recristallisation dans l'éthanol on obtient 23,9 g de cristaux blancs fondant à 124-126 °C. On les dissout dans 200 ml de chloroforme et on ajoute lentement 150 ml d'éther éthylique saturé de chlorure d'hydrogène, à 0 °C. Après l'addition on agite encore une demi-heure et on filtre le chlorhydrate précipité sur verre fritté, on lave avec deux fois 100 ml d'éther et on sèche sous vide à 50 °C. On obtient 25,66 g de cristaux blancs qui, après recristallisation dans l'éthanol, fondent à 246-247 °C.

Exemple 32 (Benzyl-4 pipéridino)-2 (chloro-2 phényl)-1 propanol et son chlorhydrate.

1. (Benzyl-4 pipéridino)-2 chloro-2' propiophénone.

On procède comme à l'exemple 31.1, en partant de 26,29 g de bromo-2 chloro-2' propiophénone dans 70 ml d'acétonitrile, et 37,23 g de benzyl-4 pipéridine. Après séparation du bromhydrate de benzyl-4 pipéridine, l'addition d'acide chlorhydrique ne provoque pas la précipitation du chlorhydrate de la cétone formée. On extrait alors ce dernier avec du chlorure de méthylène, on revient à la forme base en traitant la phase organique obtenue avec 100 ml d'ammoniaque à 25 %. Après lavage, séchage et évaporation on est en présence de 32,3 g d'une huile brune que l'on réduit telle quelle.

2. (Benzyl-4 pipéridino)-2 (chloro-2 phényl)-1 propanol et son chlorhydrate sous forme (±) érythro.

On procède comme à l'exemple 31.2 en partant des 32,3 g de cétone sous forme de base, dans 300 ml d'éthanol et 150 ml d'acide acétique, et 53,95 g de borohydrure de potassium. Après recristallisation dans l'éthanol le chlorhydrate final fond à 227-229 °C.

Exemple 33 (Benzyl-4 pipéridino)-2 (chloro-4 phényl)-1 éthanol et son chlorhydrate.

1. (Benzyl-4 pipéridino)-2 chloro-4' acétophénone.

On chauffe au reflux pendant 3 heures un mélange de 23,35 g (0,1 mole) de bromo-2 chloro-4' acétophénone, 17,5 g (0,1 mole) de benzyl-4 pipéridine, et 13,8 g (0,1 mole) de carbonate de potassium dans 250 ml d'éthanol anhydre, puis on laisse reposer une nuit. On sépare les minéraux par filtration, on les lave à l'éthanol, et on évapore le filtrat sous vide. Il reste un résidu huileux brun que l'on triture dans de l'éther éthylique. Il se forme un solide que l'on sépare par filtration puis lave à l'éther. On recueille le filtrat et on triture le solide dans un mélange de soude diluée et d'acétate d'éthyle. On sépare la phase organique, on la lave, on la sèche et on la réunit au filtrat éthéré précédemment obtenu. Après évaporation sous vide du mélange il reste une huile qui cristallise au repos et qu'on utilise telle quelle.

2. (Benzyl-4 pipéridino)-2 (chloro-4 phényl)-1 éthanol et son chlorhydrate.

On dilue l'huile précédemment obtenue par 250 ml d'éthanol et 125 ml d'acide acétique et, tout en refroidissant entre 10 et 15 °C, on ajoute lentement 12,5 g de borohydrure de potassium. Après addition on agite encore 1 heure et demie à froid puis 1 heure à température ambiante, puis on verse le mélange dans 800 ml d'eau et on ajoute de l'ammoniaque concentrée. On extrait deux fois par du chlorure de méthylène, on réunit les phases organiques, on lave à l'eau, on les sèche et on les évapore. Il reste 25,3 g d'un solide orangé que l'on triture dans de l'éther ; on obtient ainsi 14,4 g d'un solide beige. On en prépare le chlorhydrate en le chauffant dans 500 ml de méthanol et en ajoutant 435 ml de solution d'acide chlorhydrique 0,1 N dans l'alcool isopropylique. Après refroidissement, évaporation sous vide et recristallisation dans 250 ml d'alcool isopropylique, le chlorhydrate fond à 190-191 °C.

Exemple 34 (Benzyl-4 pipéridino)-2 (méthyl-2 phényl)-1 propanol et son chlorhydrate.

1. (Benzyl-4 pipéridino)-2 méthyl-2' propiophénone.

A température ambiante on agite pendant 4 heures un mélange de 25 g de bromo-2 méthyl-2' propiophénone, 70 ml d'acétonitrile sec, 30 g de carbonate de potassium et 19 g de benzyl-4 pipéridine. On filtre pour séparer le résidu minéral et on dilue le filtrat avec 200 ml d'éther éthylique, ce qui provoque la précipitation du bromhydrate de benzyl-4 pipéridine. On sépare ce dernier par filtration ; le filtrat laisse déposer un deuxième jet de bromhydrate qu'on sépare également. On acidifie alors le filtrat par 300 ml d'acide chlorhydrique 3N. Le chlorhydrate de la propiophénone intermédiaire est une huile insoluble dans les deux phases. On extrait la phase aqueuse acide deux fois par du chlorure de méthylène, on lave l'extrait à l'eau, et on lui ajoute de l'ammoniaque. On agite, on laisse décanter, on sèche la phase organique, on la filtre, on la traite par environ 100 g de silice pour retenir les traces de benzyl-4 pipéridine, on filtre et on évapore le filtrat. On recueille 10,9 g d'une huile.

2. (Benzyl-4 pipéridino)-2 (méthyl-2 phényl)-1 propanol et son chlorhydrate sous forme (±) érythro.

On dilue l'huile précédemment obtenue par 100 ml d'éthanol et 20 ml d'acide acétique, et tout en agitant sur bain de glace, on ajoute 5 g de borohydrure de sodium broyé, en l'espace d'une demi heure. Après 4 heures on ajoute progressivement 200 ml d'acide chlorhydrique 3N. Il se forme un précipité qu'on laisse reposer une nuit à température ambiante. On essore le précipité, on le lave à l'eau et on le recristallise deux fois dans l'alcool isopropylique. On en isole 5,9 g qui fondent à 243-245 °C.

Exemple 35 (Benzyl-4 pipéridino)-2 (hydroxy-4 méthyl-3 phényl)-1 propanol et son chlorhydrate.

1. (Benzyl-4 pipéridino)-2 hdyroxy-4' méthyl-3' propiophénone.

On place 12,15 g (0,05 mole) de bromo-2 hydroxy-4′ méthyl-3′ propiophénone en solution dans 25 ml d'éthanol, on ajoute 8,75 g (0,05 mole) de benzyl-4 pipéridine dilués dans 25 ml d'éthanol, puis 5,4 g (0,05 mole) de carbonate de sodium, et on chauffe au reflux pendant 1 heure et demie. On chasse le solvant et on chromatographie le résidu sur 100 g de silice en éluant avec de l'acétone. On obtient 18,4 g d'une huile épaisse qu'on utilise telle quelle.

2. (Benzyl-4 pipéridino)-2 (hydroxy-4 méthyl-3 phényl)-1 propiophénone et son chlorhydrate sous forme (±) érythro.

On dissout 8,6 g (0,025 mole) de l'huile précédemment obtenue dans 86 ml d'éthanol, on ajoute 43 ml d'acide acétique puis, par petites fractions, 14 g de borohydrure de potassium. On laisse agir une nuit puis on ajoute lentement 375 ml d'eau et 25 ml d'acétate d'éthyle. On ajoute 45 ml d'ammoniaque, on agite, on ajoute encore 2 fois 100 ml d'acétate d'éthyle. On décante la phase organique, on la lave à l'eau et on la sèche sur sulfate de sodium. On l'évapore et on obtient 9 g d'une huile que l'on purifie par chromatographie sur 500 g de silice en éluant d'abord avec un mélange 90/10 d'éther isopropylique/méthanol puis, après passage des impuretés, avec un mélange 50/50 d'éther isopropylique/méthanol. On dissout l'huile obtenue dans 40 ml d'acétate d'éthyle, on élimine un louche minéral par filtration et on ajoute 40 ml d'acide chlorhydrique aqueux 1N. On agite une heure à température ambiante, on filtre le précipité, on le lave avec de l'acide chlorhydrique puis avec 20 ml d'acétate d'éthyle, on filtre et on sèche le produit. On isole 3,8 g de chlorhydrate fondant à 200-201 °C.

Exemple 36 [(Méthyl-4 benzyl)-4 pipéridino]-2 (diméthoxy-3,4 phényl)-1 propanol et son chlorhydrate.

A un mélange de 13,65 g (0,05 mole) de bromo-2 diméthoxy-3′,4′ propiophénone et 9,46 g (0,05 mole) de (méthyl-4 benzyl)-4 pipéridine dans 50 ml d'éthanol on ajoute 5,4 g (0,05 mole) de carbonate de sodium et on chauffe au reflux pendant 3 heures. Ensuite on évapore à sec et on fait passer le résidu sur colonne de silice en éluant avec de l'acétone. On obtient une huile qu'on utilise telle quelle.

2. [(Méthyl-4 benzyl)-4 pipéridino]-2 (diméthoxy-3,4 phényl)-1 propanol et son chlorhydrate sous forme (±) érythro.

On dissout l'huile obtenue ci-dessus dans 190 ml d'éthanol, on y ajoute 95 ml d'acide acétique puis, par petites fractions, 19 g de borohydrure de potassium, et on laisse agir une nuit à température ambiante. On ajoute alors 150 ml d'acétate d'éthyle, 750 ml d'eau et 200 ml d'ammoniaque concentrée. On sépare la phase organique, on extrait la phase aqueuse encore avec 100 ml d'acétate d'éthyle, on lave les phases organiques réunies, on les sèche et on les évapore. On dissout le résidu huileux dans 80 ml d'acétate d'éthyle, on ajoute 50 ml d'acide chlorhydrique aqueux 1N, on agite le chlorhydrate précipité pendant 1 heure, on le filtre et on le recristallise dans 330 ml d'éthanol à 0,5 % d'acide chlorhydrique concentré. On obtient finalement 15 g de cristaux qui fondent à 244-245 °C.

Exemple 37 (Benzyl-4 pipéridino)-2 (dichloro-3,4 phényl)-1 propanol et son chlorhydrate.

1. (Benzyl-4 pipéridino)-2 dichloro-3′,4′ propiophénone.

On place dans un ballon 15 g de dichloro-3′,4′ propiophénone avec 100 ml de chloroforme, on ajoute une pincée de chlorure d'aluminium puis, lentement, 3,7 ml de brome dilués dans 10 ml de chloroforme. On agite le mélange une nuit à température ambiante, on filtre le mélange, et on chasse le solvant du filtat. A l'huile obtenue on ajoute 100 ml d'éthanol, 12,9 g de benzyl-4 pipéridine et 10,2 g de carbonate de potassium. On chauffe au reflux pendant 2 heures, on filtre, on chasse le solvant du filtrat, et on recueille 23,9 g d'une huile qu'on utilise telle quelle.

2. (Benzyl-4 pipéridino)-2 (dichloro-3,4 phényl)-1 propanol et son chlorhydrate sous forme (±) érythro.

A l'huile obtenue ci-dessus on ajoute 250 ml d'éthanol, 125 ml d'acide acétique et, en l'espace de 15 minutes, 16 g de borohydrure de potassium. On agite une heure sur bain de glace, puis on ajoute 600 ml d'eau glacée et on basifie avec de l'ammoniaque. On extrait avec trois fois 200 ml de chlorure de méthylène, on sèche la phase organique et on évapore le solvant. On obtient 24,3 g d'une huile que l'on dissout dans 250 ml d'éthanol et on y ajoute 10 ml d'éthanol saturé de chlorure d'hydrogène. On essore le précipité formé et on le recristallise dans l'éthanol. Le chlorhydrate obtenu fond à 226-227 °C en se décomposant.

Exemple 38 (Benzyl-4 pipéridino)-2 (diméthyl-3,5 phényl)-1 propanol et son chlorhydrate.

1. (Benzyl-4 pipéridino)-2 diméthyl-3′,5′ propiophénone.

On place 21 g de bromo-2 diméthyl-3',5' propiophénone dans 50 ml d'acétonitrile, on refroidit à 0 °C, on ajoute 30,5 g de benzyl-4 pipéridine et on agite pendant 3 heures. On dilue avec 30 ml d'éther éthylique, on sépare le bromhydrate précipité par filtration, on ajoute 150 ml d'éther au filtrat, ce qui provoque un deuxième jet de précipité. On le sépare par filtration et traite le filtrat par deux fois 200 ml d'acide chlorhydrique 3N. On sépare la phase éthérée. La phase aqueuse contient le chlorhydrate de la propiophénone formée, sous forme d'émulsion. On extrait cette phase aqueuse avec du chlorure de méthylène, on lave la phase organique obtenue avec de l'eau, on lui ajoute de l'ammoniaque diluée, on décante, on lave la phase organique à l'eau, on la sèche, on la filtre, on la traite par 100 g de silice, on filtre et on évapore le filtrat. Il reste 14,4 g d'une huile.

2. (Benzyl-4 pipéridino)-2 (diméthyl-3,5 phényl)-1 propanol et son chlorhydrate sous forme (±) érythro.

On dissout l'huile obtenue ci-dessus dans 200 ml d'éthanol, on refroidit sur bain de glace, on ajoute 40 ml d'acide acétique puis, par petites fractions, 6,5 g de borohydrure de sodium. Après 1 heure d'agitation supplémentaire on dilue le mélange avec de l'acide chlorhydrique 3N, on agite le précipité une nuit, on l'essore, on le recristallise deux fois dans l'éthanol. On récupère 7,6 g de cristaux fondant à 262-264 °C.

Exemple 39 [(Fluoro-4 benzyl)-4 pipéridino]-2 (hydroxy-4 diméthyl-3,5 phényl)-1 propanol et son chlorhydrate.

1. [(Fluoro-4 benzyl)-4 pipéridino]-2 hydroxy-4' diméthyl-3',5' propiophénone.

A un mélange de 5,14 g (0,02 mole) de bromo-2 hydroxy-4' diméthyl-3',5' propiophénone, et 4 g (0,02 mole) de (fluoro-4 benzyl)-4 pipéridine dans 25 ml d'éthanol on ajoute 2,12 g (0,02 mole) de carbonate de sodium et on chauffe au reflux pendant 3 heures. On abandonne le mélange une nuit, puis on le purifie par chromatographie sur colonne de silice en éluant avec de l'acétone. On obtient ainsi 6,6 g d'une huile brune que l'on utilise telle quelle.

2. [(Fluoro-4 benzyl)-4 pipéridino]-2 (hydroxy-4 diméthyl-3,5 phényl)-1 propanol et son chlorhydrate sous forme (±) érythro.

A l'huile précédemment obtenue en solution dans 66 ml d'éthanol on ajoute 33 ml d'acide acétique puis, par petites fractions, 6,6 g de borohydrure de potassium, et on laisse réagir une nuit à température ambiante. On ajoute alors 350 ml d'eau, on basifie le mélange avec de l'ammoniaque et on l'extrait avec de l'acétate d'éthyle. Après lavage, séchage et évaporation de la phase organique on dissout le résidu huileux dans 50 ml d'acétate d'éthyle et on ajoute 10 ml d'acide chlorhydrique aqueux 1N et on agite le mélange pendant 48 heures. On sépare ensuite le précipité par filtration, on le lave avec un mélange de 10 ml d'acide chlorhydrique 1N et 30 ml d'acétate d'éthyle et on le sèche. Il reste 1,9 g de cristaux fondant à 220-221 °C.

Exemple 40 [(Méthyl-4 benzyl)-4 pipéridino]-2 (méthoxy-4 diméthyl-3,5 phényl)- propanol et son chlorhydrate.

1. [(Méthyl-4 benzyl)-4 pipéridino]-2 méthoxy-4' diméthyl-3',5' propiophénone.

On dissout 5,9 g (0,022 mole) de bromo-2 méthoxy-4' diméthyl-3',5' propiophénone dans 30 ml d'éthanol, on ajoute 4,2 g (0,022 mole) de (méthyl-4 benzyl)-4 pipéridine, puis 2,12 g (0.02 mole) de carbonate de sodium et on chauffe au reflux pendant 2 heures. On laisse refroidir, on filtre le précipité minéral et on évapore le filtrat. On purifie l'huile résiduelle par chromatographie sur silice en éluant avec de l'acétone. On obtient 8 g de produit qu'on utilise tel quel.

2. [(Méthyl-4 benzyl)-4 pipéridino]-2 (méthoxy-4 diméthyl-3,5 phényl)-1 propanol et son chlorhydrate sous forme (±) érythro.

On dissout l'huile obtenue ci-dessus dans 80 ml d'éthanol et 20 ml d'acide acétique, on ajoute 0,8 g d'oxyde de platine et on effectue une hydrogénation dans un appareil de Parr à 50 °C et sous une pression de 0,35 MPa pendant 3 heures. Ensuite on filtre le catalyseur, on chasse le solvant du filtrat, on reprend l'huile résiduelle par de l'eau, on ajoute 10 ml d'acétate d'éthyle, on basifie le mélange avec de l'ammoniaque concentrée, et on agite 15 minutes. On sépare le précipité par filtration, on le lave à l'eau, on le dissout dans 120 ml d'éthanol chaud et on ajoute 75 ml d'acide chlorhydrique aqueux 1N. On filtre à chaud et on laisse refroidir. Le chlorhydrate cristallise. On le sépare et on le recristallise dans 100 ml d'un mélange 3/1 d'éthanol/acide chlorhydrique 1N. Après séchage il reste 3,67 g de cristaux qui fondent à

255-257 °C.

Exemple 41 (Benzyl-4 pipéridino)-2 (méthoxy-4 phényl)-1 propanol.

On agite pendant 6 heures sous azote 76,1 g de (benzyl-4 pipéridino)-2 (hydroxy-4 phényl)-1 propanol et 12,65 g de méthylate de sodium dans 1,5 l de méthanol, on évapore à sec et on reprend le sel de sodium par 1,5 l de diméthylformamide. On ajoute alors 14,74 g de sulfate de diméthyle, on agite 12 heures et on laisse reposer une nuit. On ajoute 2 l d'eau, on filtre, on extrait le filtrat par de l'éther éthylique, on lave les phases organiques, on les sèche, on les évapore et on purifie le résidu brut par chromatographie sur 200 g d'alumine en éluant avec du chlorure de méthylène. Après lavage par un peu d'éther éthylique et séchage il reste 20 g de cristaux qui fondent à 131-132 °C.

Exemple 42 (Benzyl-4 pipéridino)-2 (éthyl-4 phényl)-1 propanol et son chlorhydrate.

1. (Benzyl-4 pipéridino)-2 éthyl-4' propiophénone.

On chauffe au reflux pendant 4 heures tout en agitant, un mélange de 17,2 g de bromo-2 éthyl-4' propiophénone, 12,5 g de benzyl-4 pipéridine et 9,9 g de carbonate de potassium dans 250 ml d'éthanol sec. On sépare le produit minéral par filtration, on le lave à l'éthanol et on concentre la phase alcoolique sous vide. On reprend le résidu huileux par de l'éther éthylique et on l'extrait deux fois par de l'acide chlorhydrique dilué. Puis on extrait la phase aqueuse acide, après l'avoir nettement alcalinisée avec de la soude, trois fois à l'acétate d'éthyle. On réunit les phases organiques, on les lave à l'eau jusqu'à neutralité, on les sèche et on les évapore sous vide. On obtient 19,15 g d'un résidu huileux.

2. (Benzyl-4 pipéridino)-2 (éthyl-4 phényl)-1 propanol et son chlorhydrate sous forme (±) érythro.

Dans un erlenmeyer de 2 litres on place 19 g de la cétone huileuse précédemment obtenue en solution dans 500 ml d'éthanol sec et 150 ml d'acide acétique, et on refroidit en agitant sur bain de glace. On ajoute par petites fractions 6,1 g de borohydrure de potassium, de façon à maintenir la température inférieure à 10 °C. On agite encore une demi-heure à froid puis 2 heures à température ambiante. Puis on ajoute 500 ml d'eau et on abandonne le mélange une nuit. On alcalinise ensuite la phase homogène avec de l'ammoniaque à 28 % et on l'extrait deux fois au chlorure de méthylène. On réunit, lave, sèche et évapore les phases organiques. Il reste 18,5 g d'un solide blanchâtre. Ensuite on dissout celui-ci dans le minimum de méthanol chaud, on ajoute 550 ml de solution 0,1N d'acide chlorhydrique dans l'alcool isopropylique, et on agite en laissant refroidir jusqu'à homogénéisation complète. En concentrant le mélange à sec, on recueille un solide beige qu'on recristallise dans l'alcool isopropylique. On isole finalement 12 g de chlorhydrate pur qui fond à 239-240 °C.

Exemple 43 [(Méthyl-4 benzyl)-4 pipéridino]-2 (benzyloxy-4 phényl)-1 propanol et son chlorhydrate.

1.[(Méthyl-4 benzyl)-4 pipéridino]-2 benzyloxy-4' propiophénone.

On ajoute 13,7 g (0,05 mole) de chloro-2 (benzyloxy-4 phényl)-4' propiophénone à une solution de 9,4 g (0,05 mole) de (méthyl-4 benzyl)-4 pipéridine en solution dans 50 ml d'éthanol, puis on ajoute 5,3 g (0,05 mole) de carbonate de sodium, on chauffe au reflux pendant 3 heures et demie et on abandonne le mélange une nuit. On sépare le précipité minéral par filtration, on évapore l'éthanol, on reprend le résidu huileux par de l'acétone, et on évapore l'acétone. On chromatographie l'huile résiduelle sur 80 g de silice en éluant avec de l'acétone et on recueille 21 g d'une huile que l'on utilise telle quelle.

2. [(Méthyl-4 benzyl)-4 pipéridino]-2 (benzyloxy-4 phényl)-1 propanol et son chlorhydrate sous forme (±) érythro.

On dissout l'huile précédemment obtenue dans 200 ml d'éthanol, on ajoute 100 ml d'acide acétique puis, par petites fractions, 10 g de borohydrure de potassium, tout en agitant. On ajoute ensuite 750 ml d'eau, puis 50 ml d'acétate d'éthyle, puis 70 ml d'ammoniaque concentrée, on agite une demi-heure, on filtre le précipité, on le lave à l'eau, on le reprend par 700 ml d'éthanol à 5 % d'acide chlorhydrique concentré, on chauffe au reflux, on filtre la solution obtenue et on la laisse refroidir, ce qui provoque la précipitation du chlorhydrate. On filtre ce dernier et on le sèche. On isole ainsi 13,3 g de cristaux blancs fondant à 221-223 °C.

Exemple 44 (Benzyl-4 pipéridino)-2 (benzoyloxy-4 phényl)-1 propanol.

1. (Benzyl-4 pipéridino)-2 hydroxy-4' propiophénone.

On débenzyle 25 g de (benzyl-4 pipéridino)-2 benzyloxy-4' propiophénone dans 200 ml d'éthanol et 10

ml d'acide acétique dans un appareil de Parr en présence de 1 g de charbon palladié à 10 % sous une pression de 0,1 MPa pendant 3 heures. On isole le produit obtenu en séparant le catalyseur par filtration et en évaporant le solvant.

2. (Benzyl-4 pipéridino)-2 benzoyloxy-4' propiophénone.

A 15 ml de pyridine contenant 6 g du produit précédemment obtenu on ajoute 2,36 ml de chlorure de benzoyle, on agite le précipité formé pendant 3 heures, on le dilue à l'éther éthylique, on l'essore, on le rince à l'éther; on le sèche, on le lave trois fois à l'eau et on le sèche. On en recueille 6,8 g.

3. (Benzyl-4 pipéridino)-2 (benzoyloxy-4 phényl)-1 propanol sous forme (±) érythro.

On agite 6,7 g du produit précédemment obtenu dans 120 ml d'éthanol et 25 ml d'acide acétique sur un bain de glace, on ajoute par petites fractions 3 g de borohydrure de sodium, et on laisse agiter pendant 2 heures. On dilue à l'eau, on rend le mélange basique avec de l'ammoniaque à 7 %, on l'extrait par du chlorure de méthylène, on lave, sèche et évapore les phases organiques réunies. On triture le solide obtenu dans de l'éther, et on le recristallise dans l'éthanol. Après séchage il reste 4 g de cristaux fondant à 152-154 °C.

Exemple 45 (Benzyl-4-pipéridino)-2 (palmitoyloxy-4 phényl)-1 propanol.

1. (Benzyl-4 pipéridino)-2 palmitoyloxy-4' propiophénone.

A 40 ml de pyridine contenant 7 g de (benzyl-4 pipéridino)-2 hydroxy-4' propiophénone on ajoute 6,5 g de chlorure de palmitoyle et on agite pendant 5 heures. On dilue avec 100 ml d'éther éthylique et on abandonne une nuit. Ensuite on recueille le précipité par filtration, on le lave, on le sèche. On isole ainsi 11 g d'un produit qui fond à 185-192 °C avec décomposition.

2. (Benzyl-4 pipéridino)-2 (palmitoyloxy-4 phényl)-1 propanol sous forme (±) érythro.

On met en suspension 10 g du palmitate précédemment obtenu dans 200 ml d'éthanol et 20 ml d'acide acétique, on agite, on refroidit le mélange sur bain de glace, on ajoute par petites portions 5 g de borohydrure de sodium, on laisse agiter 5 heures et on laisse reposer une nuit à température ambiante. On sépare le produit par filtration, on l'agite dans un mélange d'eau, d'acétate d'éthyle et d'ammoniaque, on laisse décanter, on lave, on sèche et on évapore la phase organique, on triture le solide blanc obtenu dans l'éther isopropylique, on l'essore, on le recristallise dans l'acétate d'éthyle et on le sèche. On isole 5,7 g de produit qui fond à 75-77 °C.

Exemple 46 (Benzyl-4 pipéridino)-2 (hydroxy-4 hydroxyméthyl-3 phényl)-1 propanol et son fumarate neutre.

1. Benzyloxy-4' méthoxycarbonyl-3' propiophénone.

A 20,8 g d'hydroxy-4' méthoxycarbonyl-3' propiophénone dans 100 ml de diméthylformamide on ajoute 38 g de carbonate de potassium et 12,65 ml, soit 14 g de chlorure de benzyle. Tout en agitant on chauffe à 60 °C pendant 4 heures. Ensuite on jette le mélange sur de l'eau glacée, on l'extrait par de l'acétate d'éthyle, on lave la phase organique à l'eau, on la sèche, on l'évapore à sec. L'huile résiduelle cristallise par trituration dans le pentane. On filtre les cristaux, et on les recristallise dans l'éthanol. On recueille 18 g de cristaux fondant à 76-78 °C.

2. (Benzyloxy-4 méthoxycarbonyl-3 phényl)-2 éthyl-2 dioxolanne-1,3.

On chauffe au reflux pendant 10 heures un mélange de 15 g de la propiophénone préparée ci-dessus, 6,4 ml d'éthylèneglycol et 1 g d'acide p-toluènesulfonique dans 200 ml de toluène. Puis on lave la phase toluénique par 500 ml d'une solution de carbonate de sodium à 5 %, puis par de l'eau, on la sèche sur sulfate de sodium et on l'évapore à sec. On chromatographie l'huile résiduelle sur alumine en éluant avec du toluène. On isole 11 g d'huile purifiée.

3. (Benzyloxy-4 hydroxyméthyl-3 phényl)-2 éthyl-2 dioxolanne-1,3.

A une suspension de 1,2 g d'hydrure d'aluminium et de lithium dans 75 ml d'éther éthylique on ajoute goutte à goutte, à température ambiante, 11 g du dioxolanne précédemment préparé, dissous dans 75 ml d'éther éthylique, puis on chauffe au reflux pendant 1 heure. On détruit l'excès d'hydrure par addition de 2,2 ml d'eau et 1,2 ml de soude 2,5N, on filtre l'insoluble, on sèche la phase éthérée sur sulfate de sodium et on l'évapore à sec. On recueille 9,2 g d'une huile que l'on purifie sur alumine en éluant avec un

mélange 80/20 de toluène/chloroforme

4. Benzyloxy-4′ hydroxyméthyl-3′ propiophénone.

On chauffe à 60 °C pendant une demi-heure un mélange de 9 g du dioxolanne préparé comme indiqué ci-dessus, et 150 ml d'acide chlorhydrique 3N. On laisse refroidir et on extrait le mélange par de l'acétate d'éthyle, on lave, sèche et évapore la phase organique. Après recristallisation du résidu dans le benzène il reste 6 g de cristaux fondant à 95-96 °C.

5. Bromo-2 benzyloxy-4′ hydroxyméthyl-3′ propiophénone.

Dans un erlenmeyer de 500 ml on place 200 ml de tétrahydrofuranne et 6 g de la propiophénone précédemment préparée. On ajoute 2,07 g (1,84 ml) de pyrrolidinone et 12,05 g d'hydrotribromure de pyrrolidinone, on porte à reflux pendant 1 heure. Il se forme un précipité blanchâtre qu'on sépare par filtration, on évapore le filtrat à sec, on reprend l'huile résiduelle par du chloroforme, on lave, sèche et évapore la phase organique ainsi obtenue, qui cristallise dans un mélange de toluène et de pentane. On isole ainsi 7 g d'un produit qui fond à 80 °C.

6. (Benzyl-4 pipéridino)-2 benzyloxy-4′ hydroxyméthyl-3′ propiophénone.

On chauffe à reflux pendant 3 heures un mélange de 7 g de la propiophénone bromée et de 7 g de benzyl-4 pipéridine dans 200 ml d'acétonitrile, on refroidit et on ajoute 500 ml d'éther éthylique. Le bromhydrate de benzylpipéridine précipité, on le filtre, on évapore le filtrat à sec, on reprend le résidu huileux par de l'acide chlorhydrique 4N et on extrait le mélange par de l'éther éthylique. Le chlorhydrate descend sous forme d'une troisième phase huileuse. On extrait trois fois par 200 ml d'éther. On aspire la phase éthérée par dépression, on répète trois fois l'opération avec 200 ml d'éther, puis on extrait l'huile résiduelle par du chloroforme, on décante, on lave à l'eau, on sèche et on évapore à sec la phase chloroformique. On recueille 9 g de chlorhydrate.

7. (Benzyl-4 pipéridino)-2 (hydroxyméthyl-3 phényl)-1 propanol et son fumarate neutre sous forme (±) érythro.

Un mélange de 200 ml de méthanol et 50 ml d'acide acétique contenant 19 g de la propiophénone ci-dessus est soumis pendant 4 heures à une hydrogénation sur charbon palladié à 40 °C sous une pression de 0,28 MPa, dans un appareil de Parr. Puis on régénère le catalyseur et on répète l'hydrogénation à 40 °C. On sépare le catalyseur par filtration, on concentre le filtrat à sec, on reprend le résidu par de l'ammoniaque 3N et on extrait par de l'acétate d'éthyle. On lave, sèche et évapore la phase organique, on chromatographie l'huile résiduelle sur silice en éluant avec un mélange 90/10 de chloroforme/méthanol. On obtient 3 g de produit dont on prépare le fumarate neutre en ajoutant la quantité stœchiométrique d'acide fumarique anhydre. On isole le fumarate par filtration et on le recristallise dans l'éthanol. On en obtient 2,0 g qui fondent à 208-210 °C.

Exemple 47 (Benzyl-4 pipéridino)-2 (hydroxy-4 méthoxycarbonyl-3 phényl)-1 propanol et son chlorhydrate.

1. (Benzyl-4 pipéridino)-2 hydroxy-4′ méthoxycarbonyl-3′ propiophénone.

On chauffe au reflux pendant 4 heures 300 ml de méthyléthylcétone contenant 57,4 g de bromo-2 hydroxy-4′ méthoxycarbonyl-3′ propiophénone, 35 g de benzyl-4 pipéridine et 30,6 g de carbonate de potassium. On filtre à chaud le précipité, on évapore le filtrat à sec, on le reprend par de l'acide chlorhydrique 3N et on extrait par de l'éther éthylique. On agite la phase éthérée pendant une heure ; le chlorhydrate cristallise, on l'essore, on le rince à l'éther, et on le transvase dans un bécher contenant de l'eau alcalinisée par de l'ammoniaque 3N et on extrait la base ainsi libérée par de l'acétate d'éthyle. Après lavage, séchage et évaporation de la phase organique on est en présence de 52 g de produit qu'on utilise tel quel.

2. (Benzyl-4 pipéridino)-2 (hydroxy-4 méthoxycarbonyl-3 phényl)-1 propanol et son chlorhydrate sous forme (±) érythro.

Dans un erlenmeyer de 2 litres on place 47,6 g de la propiophénone ci-dessus avec 500 ml de méthanol et 250 ml d'acide acétique. On refroidit le mélange sur glace et on ajoute par petites portions 50 g de borohydrure de potassium. On laisse agiter 3 heures puis reposer une nuit. On ajoute de l'eau glacée, on alcalinise avec de l'ammoniaque 3N et on extrait le mélange par de l'acétate d'éthyle. On lave la phase organique, on sépare un précipité par filtration et on le met de côté, on décante la phase organique, on la sèche, on l'évapore, on dissout le résidu dans du méthanol, on ajoute un excès d'éther

chlorhydrique 2,9N, on sépare le chlorhydrate qui précipite et on le sèche. On prépare de la même manière le chlorhydrate à partir du précipité précédemment mis de côté. On réunit les deux jets de chlorhydrate et on les recristallise dans le méthanol. On obtient ainsi 25 g de cristaux qui fondent à 228-230 °C.

Exemple 48 (Benzyl-4 pipéridino)-2 (hydroxy-4 carbamoyl-3 phényl)-1 propanol et son chlorhydrate.

Dans un flacon à pression on place 7 g de chlorhydrate de (benzyl-4 pipéridino)-2 (hydroxy-4 méthoxycarbonyl-3 phényl)-1 propanol en solution dans 200 ml de méthanol, on ajoute un morceau d'environ 0,2 g de sodium et on fait barboter de l'ammoniac jusqu'à saturation. On laisse sous agitation pendant 4 jours puis on évapore le mélange à sec. On reprend le résidu par de l'alcool isopropylique, on ajoute du propanol chlorhydrique, et on recristallise le chlorhydrate dans de l'éthanol. On en isole 3,8 g qui fondent à 215-217 °C.

Exemple 49 [(Fluoro-4 benzyl)-4 pipéridino]-2 (chloro-4 phényl)-1 éthanol et son chlorhydrate.

1. [(Fluoro-4 benzyl)-4 pipéridino]-2 chloro-4' acétophénone.

Dans un erlenmeyer de 250 ml on place 78 ml d'éthanol auxquels on ajoute 2 ml d'une solution méthanolique de méthylate de sodium titrant 5,3 N. On ajoute 2,29 g (0,01 mole) de chlorhydrate de (fluoro-4 benzyl)-4 pipéridine et on agite 15 minutes. On ajoute ensuite 1,38 g de carbonate de potassium, puis 2,33 g (0,01 mole) de bromo-2 chloro-4' acétophénone, et on chauffe au reflux pendant 2 heures. On laisse refroidir on sépare le précipité minéral par filtration, on évapore le filtrat à sec, et on reprend l'huile résiduelle par de l'acide chlorhydrique 1N. On extrait par du chloroforme, on lave la phase organique à l'eau, on la sèche sur sulfate de sodium et on l'évapore à sec. Il reste 3,22 g de chlorhydrate brut sous forme d'huile, que l'on utilise tel quel, mais que l'on peut faire cristalliser dans un mélange acétone-éther (F=168 °C).

2. [(Fluoro-4 benzyl)-4 pipéridino]-2 (chloro-4 phényl)-1 éthanol et son chlorhydrate.

Dans un erlenmeyer de 250 ml on place 3,22 g de l'huile précédemment obtenue, en solution dans 100 ml de méthanol. On refroidit la solution sur un mélange de glace et d'eau, et on ajoute par petites quantités 1,13 g de borohydrure de potassium, et on continue l'agitation pendant 8 heures à température ambiante. On alcalinise ensuite le mélange par de l'ammoniaque aqueuse 3N, on extrait par de l'acétate d'éthyle, on lave les extraits à l'eau, on les sèche et on les évapore à sec. On recristallise le résidu dans l'alcool isopropylique, on l'essore et le lave avec du pentane. La base obtenue fond à 126-127 °C. On en prépare le chlorhydrate en dissolvant la base dans de l'acétone et en ajoutant une quantité stœchiométrique d'éther chlorhydrique. Le chlorhydrate précipite. On l'essore et on le recristallise d'abord dans de l'alcool isopropylique, puis dans un mélange éther/éthanol. Après séchage il fond à 218-220 °C.

(Voir Tableau p. 20 et suiv.)

Les composés de l'invention ont été soumis à des essais pharmacologiques.

La toxicité (dose létale 50, DL 50) des composés à été déterminée chez des souris de souche CDI par méthode graphique.

Les DL 50 vont de 30 à 1 000 mg/kg par voie i. p. et de 100 à plus de 1 000 mg/kg par voie orale.

Les composés de l'invention ont été soumis au test de l'ischémie cérébrale complète. L'ischémie est due à un arrêt cardiaque induit par une injection intraveineuse rapide de $MgCl_2$. Dans ce test on mesure le « temps de survie », c'est-à-dire l'intervalle entre le moment de l'injection de $MgCl_2$ et le dernier mouvement respiratoire observable de chaque souris. Ce dernier mouvement est considéré comme l'indice ultime d'une fonction du système nerveux central.

L'arrêt respiratoire apparaît approximativement 19 secondes après l'injection de $MgCl_2$.

Des souris mâles (Charles River CDI) sont étudiées par groupes de 10.

Les souris sont nourries et abreuvées *ad libitum* avant les essais. Le temps de survie est mesuré 10 minutes après d'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.

Les rapports entre les modifications dans le terme de survie et la dose du composé sont enregistrés graphiquement selon une courbe semilogarithmique.

Cette courbe permet le calcul de la dose effective 3 secondes ($DE_{3''}$), c'est à dire la dose (en mg/kg) qui produit une augmentation de 3 secondes du temps de survie par rapport au groupe témoin de 10 souris non traitées.

Tableau

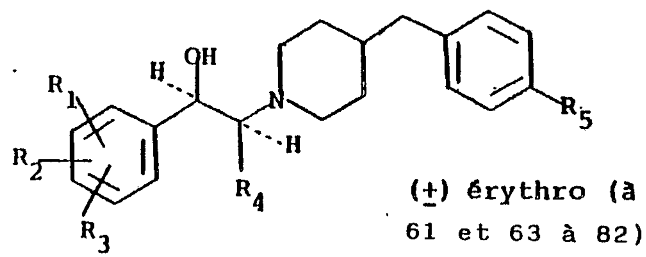

(I)

(±) érythro (à l'exception des composés n° 7, 12, 61 et 63 à 82)

| N° | Exemple | $R_1$ | $R_2$ | $R_3$ | $R_4$ | $R_5$ | Sel/base | F(°C) |
|---|---|---|---|---|---|---|---|---|
| 1 | 28 | 4-$CH_3$ | H | H | $CH_3$ | H | HCl | 251-254 (d) |
| 2 | 27 | H | H | H | $CH_3$ | H | HCl | 259-262 (d) |
| 3 | 41 | 4-$OCH_3$ | H | H | $CH_3$ | H | Base | 131-132 |
| 4 | 26 | 4-OH | H | H | $CH_3$ | $CH_3$ | HCl | 223-225 |
| 5 | | H | H | H | $CH_3$ | $CH_3$ | HCl | 239-241 |
| 6 | 43 | 4-$OCH_2$- 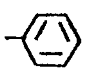 | H | H | $CH_3$ | $CH_3$ | HCl | 221-223 |
| 7 | 33 | 4-Cl | H | H | H | H | HCl | 190-191 |
| 8 | | 4-Cl | H | H | $CH_3$ | $CH_3$ | HCl | 234-235 |
| 9 | 29 | 4-Cl | H | H | $CH_3$ | $OCH_3$ | HCl | 201-202 |

Tableau (Suite)

| N° | Exemple | $R_1$ | $R_2$ | $R_3$ | $R_4$ | $R_5$ | Sel/base | F(°C) |
|---|---|---|---|---|---|---|---|---|
| 10 | | 4-F | H | H | $CH_3$ | $CH_3$ | HCl | 241-242 |
| 11 | 30 | 4-Cl | H | H | $CH_3$ | F | HCl | 211-213 |
| 12 | | 4-F | H | H | H | H | HCl | 200-201 |
| 13 | 44 | 4-OCO- 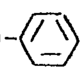 | H | H | $CH_3$ | H | Base | 152-154 |
| 14 | 45 | $4-OCOC_{15}H_{31}$ | H | H | $CH_3$ | H | Base | 75-77 |
| 15 | | $3-CH_3$ | H | H | $CH_3$ | H | HCl | 246-249 |
| 16 | 34 | $2-CH_3$ | H | H | $CH_3$ | H | HCl | 243-245 |
| 17 | | 3-Br | H | H | $CH_3$ | H | HCl | 229-230 |
| 18 | | $4-CF_3$ | H | H | $CH_3$ | H | HCl | 218-219 |
| 19 | | $3-CF_3$ | H | H | $CH_3$ | H | HCl | 254-256 |
| 20 | 31 | 3-Cl | H | H | $CH_3$ | H | HCl | 246-247 |
| 21 | | 3-F | H | H | $CH_3$ | H | HCl | 257-259 |
| 22 | | $2-CF_3$ | H | H | $CH_3$ | H | HCl | 206-208 |
| 23 | 42 | $4-C_2H_5$ | H | H | $CH_3$ | H | HCl | 239-240 |

Tableau (Suite)

| N° | Exemple | $R_1$ | $R_2$ | $R_3$ | $R_4$ | $R_5$ | Sel/base | F(°C) |
|---|---|---|---|---|---|---|---|---|
| 24 | | $4-C_2H_5$ | H | H | $CH_3$ | F | HCl | 242-243 |
| 25 | | 3-OH | H | H | $CH_3$ | H | HCl | 212-214 |
| 26 | | $3-OCH_2-$ 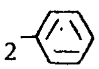 | H | H | $CH_3$ | H | HCl | 219-221 |
| 27 | | 2-F | H | H | $CH_3$ | H | HCl | 220-222 |
| 28 | 32 | 2-Cl | H | H | $CH_3$ | H | HCl | 227-229 |
| 29 | | 3-OH | H | H | $CH_3$ | $CH_3$ | HCl | 206-207 |
| 30 | 37 | 3-Cl | 4-Cl | H | $CH_3$ | H | HCl | 226-227 (d) |
| 31 | | $3-COOC_2H_5$ | 4-OH | H | $CH_3$ | H | HCl | 236-238 |
| 32 | 47 | $3-COOCH_3$ | 4-OH | H | $CH_3$ | H | HCl | 228-230 |
| 33 | 38 | $3-CH_3$ | $5-CH_3$ | H | $CH_3$ | H | HCl | 262-264 |
| 34 | 48 | $3-CONH_2$ | 4-OH | H | $CH_3$ | H | HCl | 215-217 |
| 35 | | 2-Cl | 4-Cl | H | $CH_3$ | H | HCl | > 260 (s) |
| 36 | 35 | $3-CH_3$ | 4-OH | H | $CH_3$ | H | HCl | 200-201 |
| 37 | | $3-CH_3$ | $4-OCH_3$ | H | $CH_3$ | H | HCl | 232-234 |

Tableau (Suite)

| N° | Exemple | $R_1$ | $R_2$ | $R_3$ | $R_4$ | $R_5$ | Sel/base | F(°C) |
|---|---|---|---|---|---|---|---|---|
| 38 | 36 | $3-OCH_3$ | $4-OCH_3$ | H | $CH_3$ | $CH_3$ | HCl | 244-245 |
| 39 | | 3-F | 4-OH | H | $CH_3$ | H | HCl | 218-220 |
| 40 | | $3-OCH_2-$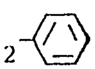 | H | H | $CH_3$ | $CH_3$ | HCl | 217-219 |
| 41 | | $3-CF_3$ | H | H | $CH_3$ | F | HCl | 254-256 (s) |
| 42 | | 3-Cl | 4-OH | H | $CH_3$ | H | HCl | 218-220 |
| 43 | | $3-COOCH_3$ | $4-OCH_3$ | H | $CH_3$ | H | HCl | 250 (d) |
| 44 | | $3-CH_3$ | $4-OCH_3$ | H | $CH_3$ | $CH_3$ | HCl | 220-222 |
| 45 | 46 | $3-CH_2OH$ | 4-OH | H | $CH_3$ | H | Fum. | 208-210 |
| 46 | | 3-F | $4-OCH_3$ | H | $CH_3$ | H | HCl | 198-200 |
| 47 | | 3-Cl | $4-OCH_3$ | H | $CH_3$ | H | HCl | 228-230 |

Tableau (Suite)

| N° | Exemple | $R_1$ | $R_2$ | $R_3$ | $R_4$ | $R_5$ | Sel/base | F(°C) |
|---|---|---|---|---|---|---|---|---|
| 48 | | 3-Cl | 5-Cl | H | $CH_3$ | H | HCl | 260-268 |
| 49 | | 3-$CH_3$ | 4-OH | 5-$CH_3$ | $CH_3$ | $CH_3$ | HCl | 237-239 |
| 50 | 40 | 3-$CH_3$ | 4-$OCH_3$ | 5-$CH_3$ | $CH_3$ | $CH_3$ | HCl | 255-257 |
| 51 | | 3-$CH_3$ | 4-OH | 5-$CH_3$ | $CH_3$ | H | HCl | 199-201 |
| 52 | 39 | 3-$CH_3$ | 4-OH | 5-$CH_3$ | $CH_3$ | F | HCl | 220-221 |
| 53 | | 4-$CH_3$ | H | H | $CH_3$ | $CH_3$ | HCl | 241-242 |
| 54 | | 4-$CH_3$ | H | H | $CH_3$ | F | HCl | 234-235 |
| 55 | | 4-$OCH_3$ | H | H | $CH_3$ | $CH_3$ | HCl | 225-227 |
| 56 | | 2-Cl | 4-Cl | H | $CH_3$ | F | HCl | 220-222 |
| 57 | | 4-Cl | H | H | $CH_3$ | 3,4,5-$(OCH_3)_3$ | HCl | 165-166 (d) |

Tableau (Suite)

| N° | Exemple | $R_1$ | $R_2$ | $R_3$ | $R_4$ | $R_5$ | Sel/base | F°(C) |
|---|---|---|---|---|---|---|---|---|
| 58 | | 3-F | 4-$OCH_3$ | H | $CH_3$ | F | HCl | 221-213 |
| 59 | | 3-Cl | 4-$OCH_3$ | H | $CH_3$ | F | HCl | 234-236 |
| 60 | 49 | 4-Cl | H | H | H | F | HCl | 218-220 |
| 61 | | 3-$OCH_3$ | H | H | $CH_3$ | H | HCl | 228-230 |
| 62 | | 4-Br | H | H | H | H | HCl | 204-205 |
| 63 | | 3-Cl | 4-Cl | H | H | H | HCl | 214-215 |
| 64 | | 2-Cl | H | H | H | H | HCl | 218-220 |
| 65 | | 3-Cl | H | H | H | H | HCl | 215-216 |
| 66 | | 3-Cl | H | H | H | F | HCl | 245-246 |
| 67 | | 4-F | H | H | H | F | HCl | 213-215 |
| 68 | | 2-Cl | H | H | H | F | HCl | 182 |
| 69 | | 3-Cl | 4-Cl | H | H | F | HCl | 236-238 |
| 70 | | 2-Br | H | H | H | F | HCl | 202-204 |
| 71 | | 3-Br | H | H | H | F | HCl | 247-248 |
| 72 | | 4-Br | H | H | H | F | HCl | 214 |
| 73 | | H | H | H | H | F | HCl | 235 |
| 74 | | 4-OH | H | H | H | F | HCl | 193-194 |

Tableau (Suite)

| N° | Exemple | $R_1$ | $R_2$ | $R_3$ | $R_4$ | $R_5$ | Sel/base | F°(C) |
|---|---|---|---|---|---|---|---|---|
| 75 | | 4-$CF_3$ | H | H | H | F | HCl | 208-210 |
| 76 | | 2-$CH_3$ | H | H | H | F | HCl | 206-207 |
| 77 | | 3-$CH_3$ | H | H | H | F | HCl | 208-209 |
| 78 | | 4-$CH_3$ | H | H | H | F | HCl | 209-210 |
| 79 | | 2-$OCH_3$ | H | H | H | F | HCl | 222-224 |
| 80 | | 3-$OCH_3$ | H | H | H | F | HCl | 202-204 |
| 81 | | 4-$OCH_3$ | H | H | H | F | HCl | 159-160 |
| 82 | | 2-Cl | H | H | Et | H | HCl | 217-218 |
| 83 | | 4-Cl | H | H | Et | H | HCl | 214-215 |

légende :
(d) : décomposition
(s) : sublimation
HCl : chlorhydrate
Fum : fumarate

Un augmentation de 3 secondes dans le temps de survie est à la fois significative statistiquement et reproductible.

Les $DE_{3''}$ des composés de l'invention vont de 5 à 100 mg/kg par voie i. p.

L'étude pharmacologique des composés de l'invention montre qu'ils possèdent une activité antianoxique et qu'ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des encéphalopathies métaboliques, et pour le traitement des états dépressifs.

L'invention comprend par conséquent, toutes compositions pharmaceutiques renfermant les composés et/ou leurs sels comme principes actifs, en association avec tous excipients appropriés à leur administration, en particulier par voie orale ou parentérale.

Les voies d'administration peuvent être les voies orale et parentérale.

La posologie quotidienne peut aller de 1 à 100 mg par voie parentérale et de 5 à 500 mg par voie orale.

**Revendications** (pour les Etats contractants : BE, CH, DE, FR, GB, IT, LI, LU, NL, SE)

1. Composés répondant à la formule générale (I),

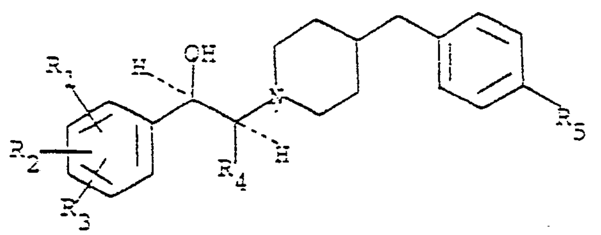

(I)

dans laquelle

$R_1$ représente un atome d'hydrogène, un atome d'halogène, un groupe trifluorométhyle, un groupe alkyle ayant de 1 à 4 atomes de carbone, un groupe hydroxyle, un groupe alcoxyle ayant de 1 à 4 atomes de carbone, un groupe benzyloxyle, un groupe alcanoyloxyle ayant de 1 à 16 atomes de carbone, un groupe benzoyloxyle ou encore, lorsque $R_2$ représente un groupe hydroxyle ou méthoxyle en position 4 et $R_3$ représente un atome d'hydrogène, $R_1$ peut représenter un groupe hydroxy-méthyle, un groupe carbamoyle, ou un groupe alcoxycarbonyle ayant de 1 à 4 atomes de carbone dans la partie alcoxy,

$R_2$ représente un atome d'hydrogène, un atome d'halogène, un groupe alkyle de 1 à 4 atomes de carbone, un groupe hydroxyle, ou un groupe alcoxyle ayant 1 à 4 atomes de carbone,

$R_3$ représente un atome d'hydrogène ou un groupe alkyle ayant 1 à 4 atomes de carbone,

$R_4$ représente un groupe alkyle ayant de 1 à 4 atomes de carbone, les composés étant alors sous forme (±) érythro, ou, lorsque $R_3$ représente un atome d'hydrogène, $R_4$ peut aussi représenter un atome d'hydrogène,

$R_5$ représente un atome d'hydrogène, un atome d'halogène, un groupe alkyle ayant 1 à 4 atomes de carbone, un groupe alcoxyle ayant 1 à 4 atomes de carbone, ou un ensemble de trois groupes méthoxyles en positions 3, 4, 5 du radical benzyle,

ainsi que leurs sels d'additions à des acides pharmaceutiquement acceptables, à l'exception des composés dans la formule (I) desquels

a) l'un des substituants $R_1$ et $R_2$ est en position 4 et représente un groupe hydroxyle, alcoxyle ou benzyloxyle, l'autre est en position 3 et représente un atome d'hydrogène ou un groupe hydroxyle, alcoxyle ou benzyloxyle, et $R_3$ et $R_5$ représentent chacun un atome d'hydrogène, ou bien

b) $R_1$ ou $R_2$ est en position 4 et représente un atome d'halogène, $R_4$ représente un groupe méthyle, et les autres substituants sont chacun un atome d'hydrogène.

2. Composés selon la revendication 1, dans la formule (I) desquels

$R_1$ représente un atome d'hydrogène, un atome d'halogène, un groupe trifluorométhyle, un groupe méthyle, un groupe hydroxyle ou un groupe méthoxyle,

$R_2$ représente un atome d'hydrogène, un atome d'halogène, un groupe méthyle, un groupe hydroxyle ou un groupe méthoxyle,

$R_3$ représente un atome d'hydrogène ou un groupe méthyle,

$R_4$ représente un atome d'hydrogène ou un groupe méthyle, et

$R_5$ représente un atome d'hydrogène, un atome d'halogène, un groupe méthyle, un groupe méthoxyle ou un ensemble de trois groupes méthoxyles en positions 3, 4, 5 du radical benzyle.

3. Composés selon la revendication 1, dans la formule (I)

$R_1$ représente un groupe hydroxyméthyle, méthoxycarbonyle, éthoxycarbonyle ou carbamoyle,

$R_2$ représente un groupe hydroxyle,

$R_3$ représente un atome d'hydrogène,
$R_4$ représente un groupe méthyle, et
$R_5$ représente un atome d'hydrogène.

4. Composés selon la revendication 1, dans la formule (I) desquels
$R_1$ représente un atome d'halogène,
$R_2$ et $R_3$ représentent chacun un atome d'hydrogène,
$R_4$ représente un atome d'hydrogène ou un groupe méthyle, et
$R_5$ représente un atome d'hydrogène ou d'halogène.

5. Le (benzyl-4 pipéridino)-2 (chloro-4 phényl)-1 éthanol et ses sels d'addition à des acides pharmaceutiquement acceptables.

6. Le (benzyl-4 pipéridino)-2 (chloro-2 phényl)-1 propanol et ses sels d'addition à des acides pharmaceutiquement acceptables.

7. Le [(fluoro-4 benzyl)-4 pipéridino]-2 (chloro-4 phényl)-1 éthanol et ses sels d'addition à des acides pharmaceutiquement acceptables.

8. Procédé de préparation des composés de formule (I) selon la revendication 1, caractérisé en ce qu'on fait réagir une cétone de formule II

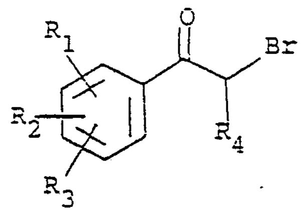

(II)

dans laquelle $R_1$, $R_2$, $R_3$ et $R_4$ sont tels que définis à la revendication 1, avec une benzylpipéridine de formule III

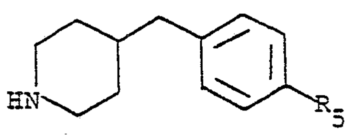

(III)

dans laquelle $R_5$ est tel que défini à la revendication 1, puis on soumet la cétone obtenue, de formule IV,

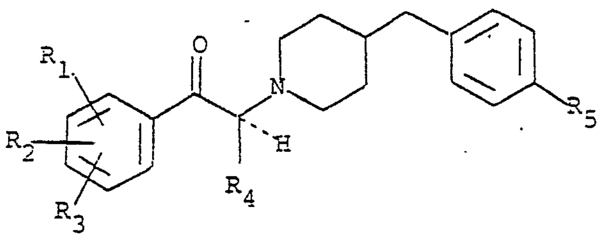

(IV)

à une réduction, éventuellement après en avoir modifié les substituants $R_1$, $R_2$ et $R_3$, et si on le désire, on modifie les substituants $R_1$, $R_2$ et $R_3$ du composé de formule I ainsi obtenu.

9. Procédé selon la revendication 8, caractérisé en ce qu'on fait réagir les composés de formules II et III dans un solvant en présence d'une base faible.

10. Procédé selon la revendication 8, caractérisé en ce qu'on fait réagir les composés de formules II et III dans l'acétonitrile, à raison de 1 mole du premier pour 2 moles du second.

11. Procédé selon la revendication 8, caractérisé en ce qu'on réduit la cétone de formule IV par une hydrogénation catalytique.

12. Procédé selon la revendication 8, caractérisé en ce qu'on réduit la cétone de formule IV par le borohydrure de sodium ou de potassium en milieu acide.

13. Composition pharmaceutique, caractérisée en ce qu'elle contient à titre de substance active un composé selon l'une des revendications 1 à 7.

**Revendications** (pour l'état contractant AT)

1. Procédé de préparation de composés répondant à la formule générale (I)

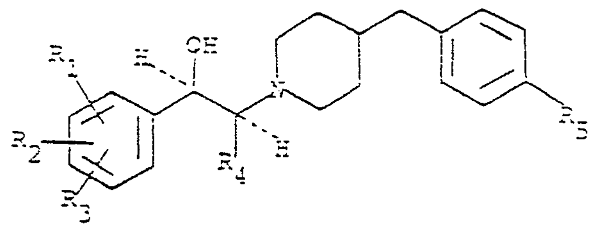

(I)

dans laquelle

$R_1$ représente un atome d'hydrogène, un atome d'halogène, un groupe trifluorométhyle, un groupe alkyle ayant de 1 à 4 atomes de carbone, un groupe hydroxyle, un groupe alcoxyle ayant de 1 à 4 atomes de carbone, un groupe benzyloxyle, un groupe alcanoyloxyle ayant de 1 à 16 atomes de carbone, un groupe benzoyloxyle ou encore, lorsque $R_2$ représente un groupe hydroxyle ou méthoxyle en position 4 et $R_3$ représente un atome d'hydrogène, $R_1$ peut représenter un groupe hydroxy-méthyle, un groupe carbamoyle, ou un groupe alcoxycarbonyle ayant de 1 à 4 atomes de carbone dans la partie alcoxy,

$R_2$ représente un atome d'hydrogène, un atome d'halogène, un groupe alkyle de 1 à 4 atomes de carbone, un groupe hydroxyle, ou un groupe alcoxyle ayant 1 à 4 atomes de carbone,

$R_3$ représente un atome d'hydrogène ou un groupe alkyle ayant 1 à 4 atomes de carbone,

$R_4$ représente un groupe alkyle ayant de 1 à 4 atomes de carbone, les composés étant alors sous forme (±) érythro, ou, lorsque $R_3$ représente un atome d'hydrogène, $R_4$ peut aussi représenter un atome d'hydrogène,

$R_5$ représente un atome d'hydrogène, un atome d'halogène, un groupe alkyle ayant 1 à 4 atomes de carbone, un groupe alcoxyle ayant 1 à 4 atomes de carbone, ou un ensemble de trois groupes méthoxyles en positions 3, 4, 5 du radical benzyle,

ainsi que leurs sels d'additions à des acides pharmaceutiquement acceptables, à l'exception des composés dans la formule (I) desquels

a) l'un des substituants $R_1$ et $R_2$ est en position 4 et représente un groupe hydroxyle, alcoxyle ou benzyloxyle, l'autre est en position 3 et représente un atome d'hydrogène ou un groupe hydroxyle, alcoxyle ou benzyloxyle, et $R_3$ et $R_5$ représentent chacun un atome d'hydrogène, ou bien

b) $R_1$ ou $R_2$ est en position 4 et représente un atome d'halogène, $R_4$ représente un groupe méthyle, et les autres substituants sont chacun un atome d'hydrogène,

procédé caractérisé en ce qu'on fait réagir une cétone de formule II

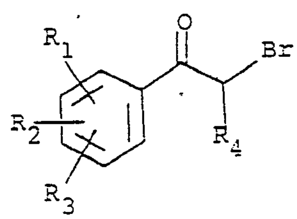

(II)

dans laquelle $R_1$, $R_2$, $R_3$ et $R_4$ sont tels que définis ci-dessus, avec une benzylpipéridine de formule III

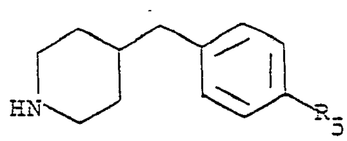

(III)

dans laquelle $R_5$ est tel que défini ci-dessus, puis on soumet la cétone obtenue, de formule IV,

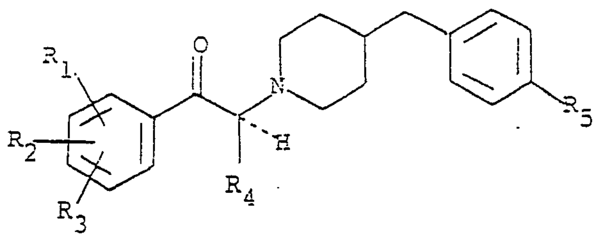

(IV)

à une réduction, éventuellement après en avoir modifié les substituants $R_1$, $R_2$ et $R_3$, et si on le désire, on modifie les substituants $R_1$, $R_2$ et $R_3$ du composé de formule I ainsi obtenu.

2. Procédé selon la revendication 1, caractérisé en ce qu'on fait réagir les composés de formules II et III dans un solvant en présence d'une base faible.

3. Procédé selon la revendication 1, caractérisé en ce qu'on fait réagir les composés de formules II et III dans l'acétonitrile, à raison de 1 mole du premier pour 2 moles du second.

4. Procédé selon la revendication 1, caractérisé en ce qu'on réduit la cétone de formule IV par une hydrogénation catalytique.

5. Procédé selon la revendication 1, caractérisé en ce qu'on réduit la cétone de formule IV par le borohydrure de sodium ou de potassium en milieu acide.

6. Procédé selon l'une quelconque des revendications 1 à 5, caractérisé en ce que, dans la formule (I),

$R_1$ représente un atome d'halogène,

$R_2$ et $R_3$ représentent chacun un atome d'hydrogène,

$R_4$ représente un atome d'hydrogène ou un groupe méthyle, et

$R_5$ représente un atome d'hydrogène ou d'halogène.

**Claims** (for the Contracting States : BE, CH, DE, FR, GB, IT, LI, LU, NL, SE)

1. Compounds corresponding to the general formula (I)

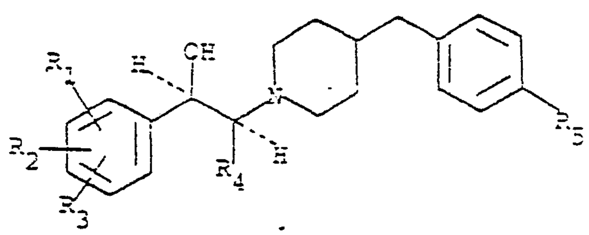

(I)

in which :

$R_1$ represents a hydrogen atom, a halogen atom, a trifluoromethyl group, an alkyl group having from 1 to 4 carbon atoms, a hydroxyl group, an alkoxy group having from 1 to 4 carbon atoms, a benzyloxy group, an alkanoyloxy group having from 1 to 16 carbon atoms or a benzoyloxy group, or, if $R_2$ represents a hydroxy or methoxyl group in the 4-position and $R_3$ represents a hydrogen atom, $R_1$ can represent a hydroxymethyl group, a carbamoyl group or an alkoxycarbonyl group having from 1 to 4 carbon atoms in the alkoxy part,

$R_2$ represents a hydrogen atom, a halogen atom, an alkyl group having from 1 to 4 carbon atoms, a hydroxyl group or an alkoxy group having from 1 to 4 carbon atoms,

$R_3$ represents a hydrogen atom or an alkyl group having from 1 to 4 carbon atoms,

$R_4$ represents an alkyl group having from 1 to 4 carbon atoms, in which case the compounds are in the (±)-erythro form, or, if $R_3$ represents a hydrogen atom, $R_4$ can also represent a hydrogen atom, and

$R_5$ represents a hydrogen atom, a halogen atom, an alkyl group having from 1 to 4 carbon atoms, an alkoxy group having from 1 to 4 carbon atoms or a set of three methoxy groups in the 3-, 4- and 5-positions of the benzyl radical,

and also their addition salts with pharmaceutically acceptable acids, with the exception of the compounds in the formula (I) of which :

a) one of the substituents $R_1$ and $R_2$ is in the 4-position and represents a hydroxyl, alkoxyl or benzyloxyl group, the other is in the 3-position and represents a hydrogen atom or a hydroxyl, alkoxyl or benzyloxyl group, and $R_3$ and $R_5$ each represent a hydrogen atom, or alternatively

b) $R_1$ or $R_2$ is in the 4-position and represents a halogen atom, $R_4$ represents a methyl group and the other substituents each represent a hydrogen atom.

2. Compounds according to claim 1 in the formula (I) of which :

$R_1$ represents a hydrogen atom, a halogen atom, a trifluoromethyl group, a methyl group, a hydroxyl group or a methoxyl group,

$R_2$ represents a hydrogen atom, a halogen atom, a methyl group, a hydroxyl group or a methoxyl group,

$R_3$ represents a hydrogen atom or a methyl group,

$R_4$ represents a hydrogen atom or a methyl group and

$R_5$ represents a hydrogen atom, a halogen atom, a methyl group, a methoxyl group or a set of three methoxyl groups in the 3-, 4- and 5-positions of the benzyl radical.

3. Compounds according to claim 1 in the formula (I) of which :

$R_1$ represents a hydroxymethyl, methoxycarbonyl, ethoxycarbonyl or carbamoyl group,

$R_2$ represents a hydroxyl group,

$R_3$ represents a hydrogen atom,
$R_4$ represents a methyl group and
$R_5$ represents a hydrogen atom.

4. Compounds according to claim 1 in the formula (I) of which :
$R_1$ represents a halogen atom,
$R_2$ and $R_3$ each represent a hydrogen atom,
$R_4$ represents a hydrogen atom or a methyl group and
$R_5$ represents a hydrogen or halogen atom.

5. 2-(4-Benzylpiperidino)-1-(4-chlorophenyl)-ethanol and its addition salts with pharmaceutically acceptable acids.

6. 2-(4-Benzylpiperidino)-1-(2-chlorophenyl)-propanol and its addition salts with pharmaceutically acceptable acids.

7. 2-[4-(4-Fluorobenzyl)-piperidino]-1-(4-chlorophenyl)-ethanol and its addition salts with pharmaceutically acceptable acids.

8. Process for the preparation of the compounds of the formula (I) according to claim 1, characterised in that a ketone of the formula II

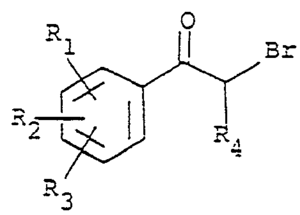

(II)

in which $R_1$, $R_2$, $R_3$ and $R_4$ are as defined in claim 1, is reacted with a benzylpiperidine of the formula III

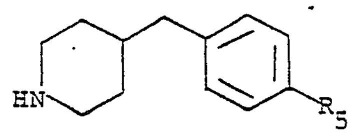

(III)

in which $R_5$ is as defined in claim 1, the resulting ketone of the formula IV

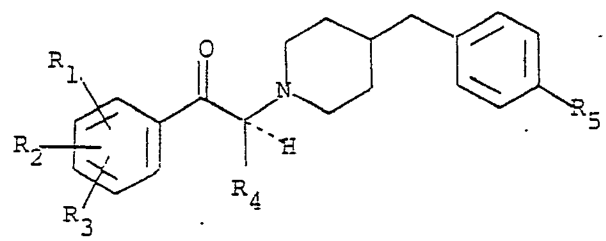

(IV)

is then subjected to reduction, if appropriate after its substituents $R_1$, $R_2$ and $R_3$ have been modified, and, if desired, the substituents $R_1$, $R_2$ and $R_3$ of the resulting compound of the formula I are modified.

9. Process according to claim 8, characterised in that the compounds of the formulae II and III are reacted in a solvent, in the presence of a weak base.

10. Process according to claim 8, characterised in that the compounds of the formulae II and III are reacted in acetonitrile, in proportions of 1 mol of the first to 2 mol of the second.

11. Process according to claim 8, characterised in that the ketone of the formula IV is reduced by catalytic hydrogenation.

12. Process according to claim 8, characterised in that the ketone of the formula IV is reduced with sodium borohydride or potassium borohydride in an acid medium.

13. Pharmaceutical composition, characterised in that it contains, as the active substance, a compound according to one of claims 1 to 7.

**Claims** (for the Contracting State AT)

1. Process for the preparation of compounds corresponding to the general formula (1)

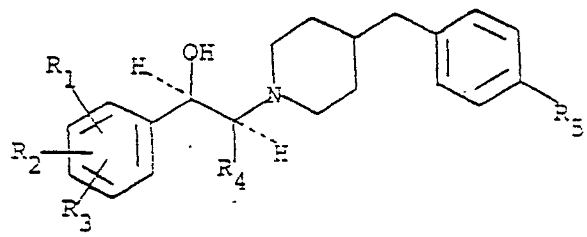

(I)

in which

$R_1$ represents a hydrogen atom, a halogen atom, a trifluoromethyl group, an alkyl group having from 1 to 4 carbon atoms, a hydroxyl group, an alkoxy group having from 1 to 4 carbon atoms, a benzyloxy group, an alkanoyloxy group having from 1 to 16 carbon atoms or a benzoyloxy group, or, if $R_2$ represents a hydroxy or methoxyl group in the 4-position and $R_3$ represents a hydrogen atom, $R_1$ can represent a hydroxymethyl group, a carbamoyl group or an alkoxycarbonyl group having from 1 to 4 carbon atoms in the alkoxy part,

$R_2$ represents a hydrogen atom, a halogen atom, an alkyl group having from 1 to 4 carbon atoms, a hydroxyl group or an alkoxyl group having from 1 to 4 carbon atoms,

$R_3$ represents a hydrogen atom or an alkyl group having from 1 to 4 carbon atoms,

$R_4$ represents an alkyl group having from 1 to 4 carbon atoms, in which case the compounds are in the (±)-erythro form, or, if $R_3$ represents a hydrogen atom, $R_4$ can also represent a hydrogen atom, and

$R_5$ represents a hydrogen atom, a halogen atom, an alkyl group having from 1 to 4 carbon atoms, an alkoxy group having from 1 to 4 carbon atoms or a set of three methoxy groups in the 3-, 4- and 5-positions of the benzyl radical,

and also their additions salts with pharmaceutically acceptable acids, with the exception of the compounds in the formula (I) of which :

a) one of the substituents $R_1$ and $R_2$ is in the 4-position and represents a hydroxyl, alkoxyl or benzyloxyl group, the other is in the 3-position and represents a hydrogen atom or a hydroxyl, alkoxyl or benzyloxyl group, and $R_3$ and $R_5$ each represent a hydrogen atom, or alternatively

b) $R_1$ or $R_2$ is in the 4-position and represents a halogen atom, $R_4$ represents a methyl group and the other substituents each represent a hydrogen atom,

process characterized in that a ketone of the formula II

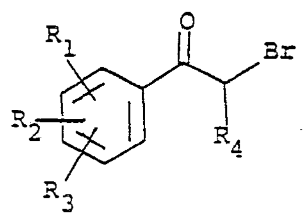

(II)

in which $R_1$, $R_2$, $R_3$ and $R_4$ are as defined in above, is reacted with a benzylpiperidine of the formula III

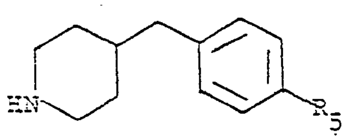

(III)

in which $R_5$ is a defined above, the resulting ketone of the formula IV

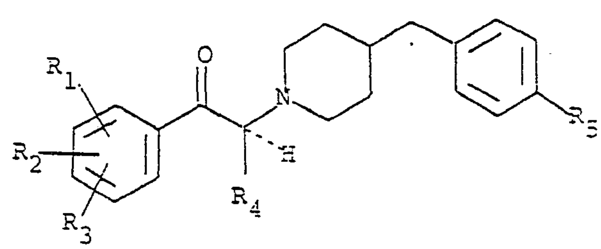

(IV)

2. Process according to claim 1, characterised in that the compounds of the formulae II and III are reacted in a solvent, in the presence of a weak base.

3. Process according to claim 1, characterised in that the compounds of the formulae II and III are reacted in acetonitrile, in proportions of 1 mol of the first to 2 mol of the second.

4. Process according to claim 1, characterised in that the ketone of the formula IV is reduced by catalytic hydrogenation.

5. Process according to claim 1, characterised in that the ketone of the formula IV is reduced with sodium borohydride or potassium borohybride in an acid medium.

6. Process according to anyone of claims 1 to 6, characterised in that, in the formula (I),

$R_1$ represents a halogen atom,

$R_2$ and $R_3$ each represent a hydrogen atom,

$R_4$ represents a hydrogen atom or a methyl group and

$R_5$ represents a hydrogen or halogen atom.

**Patentansprüche** (für die Vertragsstaaten : BE, CH, DE, FR, GB, IT, LI, LU, NL, SE)

1. Verbindung der allgemeinen Formel (I)

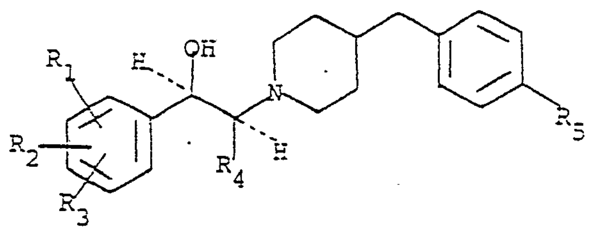

(I)

in welcher

$R_1$ ein Wasserstoffatom, ein Halogenatom, eine Trifluormethylgruppe, eine Alkylgruppe mit 1 bis 4 Kohlenstoffatomen, eine Hydroxygruppe, eine Alkoxygruppe mit 1 bis 4 Kohlenstoffatomen, eine Benzyloxygruppe, eine Alkanoyloxygruppe mit 1 bis 16 Kohlenstoffatomen, eine Bezoyloxygruppe oder, wenn $R_2$ für eine Hydroxy- oder eine Methoxygruppe in 4-Stellung steht und $R_3$ ein Wasserstoffatom bedeutet, eine Hydroxymethyl-, eine Carbamoyl- oder eine Alkoxycarbonylgruppe mit 1 bis 4 Kohlenstoffatomen im Alkoxyteil bedeutet,

$R_2$ für ein Wasserstoffatom, ein Halogenatom, eine Alkylgruppe mit 1 bis 4 Kohlenstoffatomen, eine Hydroxygruppe oder eine Alkoxygruppe mit 1 bis 4 Kohlenstoffatomen steht,

$R_3$ ein Wasserstoffatom oder eine Alkylgruppe mit 1 bis 4 Kohlenstoffatomen darstellt,

$R_4$ eine Alkylgruppe mit 1 bis 4 Kohlenstoffatomen bedeutet, wobei die Verbindungen in der (±) erythro-Form vorliegen, oder, wenn $R_3$ ein Wasserstoffatom darstellt, $R_4$ auch für ein Wasserstoffatom stehen kann,

$R_5$ für ein Wasserstoffatom, ein Halogenatom, eine Alkylgruppe mit 1 bis 4 Kohlenstoffatomen, eine Alkoxygruppe mit 1 bis 4 Kohlenstoffatomen, oder eine Gesamtheit von 3 Methoxygruppen in den Stellungen 3, 4 und 5 des Benzylrestes steht,

sowie deren Additionssalze mit pharmazeutisch verwendbaren Säuren, mit Ausnahme von Verbindungen, in deren Formel (I)

a) einer der Substituenten $R_1$ und $R_2$ in 4-Stellung steht und eine Hydroxy, Alkoxy- oder Benzyloxygruppe bedeutet, der andere in 3-Stellung steht und ein Wasserstoffatom oder eine Hydroxy-, Alkoxy- oder Benzyloxygruppe bedeutet und $R_3$ und $R_5$ jeweils für ein Wasserstoffatom stehen oder aber

b) $R_1$ oder $R_2$ in 4-Stellung stehen und ein Wasserstoffatom bedeuten, $R_4$ für eine Methylgruppe steht und die anderen Substituenten jeder ein Wasserstoffatom darstellen.

2. Verbindung nach Anspruch 1, in deren Formel (I)

$R_1$ ein Wasserstoffatom, ein Halogenatom, eine Trifluormethylgruppe, eine Methylgruppe, eine Hydroxygruppe oder eine Methoxygruppe darstellt,

$R_2$ ein Wasserstoffatom, ein Halogenatom, eine Methylgruppe, eine Hydroxygruppe oder eine Methoxygruppe bedeutet,

$R_3$ für ein Wasserstoffatom oder eine Methylgruppe steht,

$R_4$ ein Wasserstoffatom oder eine Methylgruppe bedeutet und

$R_5$ ein Wasserstoffatom, ein Halogenatom, eine Methylgruppe, Methoxygruppe oder eine Gesamtheit von 3 Methoxygruppen in den Stellungen 3,4 und 5 des Benzylrestes bedeutet.

3. Verbindungen nach Anspruch 1, in deren Formel (I)

$R_1$ eine Hydroxymethylgruppe, Methylcarbonylgruppe, Ethoxycarbonylgruppe oder Carbamoylgruppe bedeutet,

$R_2$ für eine Hydroxygruppe,

$R_3$ für ein Wasserstoffatom,

$R_4$ für eine Methylgruppe und

$R_5$ für ein Wasserstoffatom stehen.

4. Verbindungen nach Anspruch 1, in deren Formel (I) $R_1$ für ein Halogenatom, $R_2$ und $R_3$ für ein Wasserstoffatom, $R_4$ für ein Wasserstoffatom oder eine Methylgruppe und $R_5$ für ein Wasserstoff- oder ein Halogenatom stehen.

5. 2-(4-Benzylpiperidino)-1-(4-chlorphenyl)-ethanol und seine Additionssalze mit pharmazeutisch verwendbaren Säuren.

6. 2-(4-Benzylpiperidino)-1-(2-chlorphenyl)-propanol und seine Additionssalze mit pharmazeutisch verwendbaren Säuren.

7. 2-[4-(4-Fluorbenzyl)-piperidino]-1-(4-chlorphenyl)-ethanol und seine Additionssalze mit pharmazeutisch verwendbaren Säuren.

8. Verfahren zur Herstellung von Verbindungen der Formel (I) nach Anspruch 1, dadurch gekennzeichnet, daß man ein Keton der Formel II

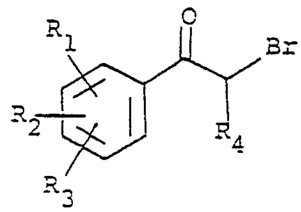

(II)

in welcher $R_1$, $R_2$, $R_3$ und $R_4$ die in Anspruch 1 angegebene Definition haben, mit einem Benzylpiperidin der Formel III

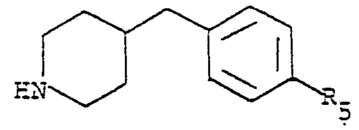

(III)

in welcher $R_5$ die in Anspruch 1 angegebene Definition hat, umsetzt, dann das erhaltene Keton der Formel IV

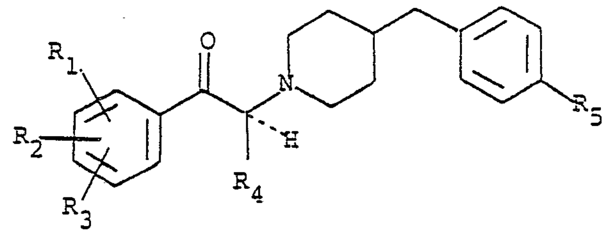

(IV)

gegebenenfalls nach Modifikation der Substituenten $R_1$, $R_2$ und $R_3$, einer Reduktion unterwirft und gewünschtenfalls die Substituenten $R_1$, $R_2$ und $R_3$ der so erhaltenen Verbindung der Formel I modifiziert.

9. Verfahren nach Anspruch 8, dadurch gekennzeichnet, daß man die Verbindungen der Formeln II und III in einem Lösungsmittel in Gegenwart einer schwachen Base reagieren läßt.

10. Verfahren nach Anspruch 8, dadurch gekennzeichnet, daß man die Verbindungen der Formeln II und III in Acetonitril in einem Verhältnis von 1 Mol des ersten zu 2 Molen des zweiten reagieren läßt.

11. Verfahren nach Anspruch 8, dadurch gekennzeichnet, daß man das Keton der Formel IV durch katalytische Hydrierung reduziert.

12. Verfahren nach Anspruch 8, dadurch gekennzeichnet, daß man das Keton der Formel IV mit Natriumborhydrid oder Kaliumborhydrid in saurem Medium reduziert.

13. Pharmazeutische Zusammensetzung, dadurch gekennzeichnet, daß sie als Wirkstoff eine Verbindung nach einem der Ansprüche 1 bis 7 enthält.

**Patentansprüche** (für den Vertragsstaat AT)

1. Verfahren zur Herstellung von Verbindungen der allgemeinen Formel (I)

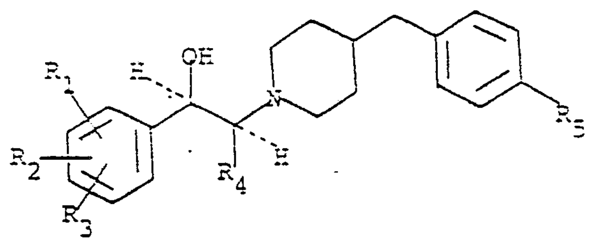

(I)

in welcher

$R_1$ ein Wasserstoffatom, ein Halogenatom, eine Trifluormethylgruppe, eine Alkylgruppe mit 1 bis 4 Kohlenstoffatomen, eine Hydroxygruppe, eine Alkoxygruppe mit 1 bis 4 Kohlenstoffatomen, eine Benzyloxygruppe, eine Alkanoyloxygruppe mit 1 bis 16 Kohlenstoffatomen, eine Bezoyloxygryppe oder, wenn $R_2$ für eine Hydroxy- oder eine Methoxygruppe in 4-Stellung steht und $R_3$ ein Wasserstoffatom bedeutet, eine Hydroxymethyl-, eine Carbamoyl- oder eine Alkoxycarbonylgruppe mit 1 bis 4 Kohlenstoffatomen im Alkoxyteil bedeutet,

$R_2$ für eine Wasserstoffatom, ein Halogenatom, eine Alkylgruppe mit 1 bis 4 Kohlenstoffatomen, eine Hydroxygruppe oder eine Alkoxygruppe mit 1 bis 4 Kohlenstoffatomen steht,

$R_3$ ein Wasserstoffatom oder eine Alkylgruppe mit 1 bis 4 Kohlenstoffatomen darstellt,

$R_4$ eine Alkylgruppe mit 1 bis 4 Kohlenstoffatomen bedeutet, wobei die Verbindungen in der (±) erythro-Form vorliegen, oder, wenn $R_3$ ein Wasserstoffatom darstellt, $R_4$ auch für ein Wasserstoffatom stehen kann,

$R_5$ für ein Wasserstoffatom, ein Halogenatom, eine Alkylgruppe mit 1 bis 4 Kohlenstoffatomen, eine Alkoxygruppe mit 1 bis 4 Kohlenstoffatomen, odereine Gesamtheit von 3 Methoxygruppen in den Stellungen 3, 4 und 5 des Benzylrestes steht,

sowie von Additionssalzen derselben mit pharmazeutisch verträglichen Säuren mit Ausnahme von Verbindungen, in deren Formel (I)

a) einer der Substituenten $R_1$ und $R_2$ in 4-Stellung steht und eine Hydroxy, Alkoxy- oder Benzyloxygruppe bedeutet, der andere in 3-Stellung steht und ein Wasserstoffatom oder eine Hydroxy-, Alkoxy- oder Benzyloxygruppe bedeutet und $R_3$ und $R_5$ jeweils für ein Wasserstoffatom stehen oder aber

b) $R_1$ oder $R_2$ in 4-Stellung stehen und ein Wasserstoffatom bedeuten, $R_4$ für eine Methylgruppe steht und die anderen Substituenten jeder ein Wasserstoffatom darstellen,

dadurch gekennzeichnet, daß man ein Keton der Formel II

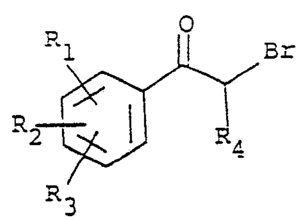

(II)

in welcher $R_1$, $R_2$, $R_3$ und $R_4$ die oben angegebenen Bedeutung haben, mit einem Benzylpiperidin der Formel III

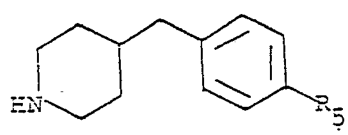

(III)

in welcher $R_5$ die oben angegebene Bedeutung hat, umsetzt, dann das erhaltene Keton der Formel IV

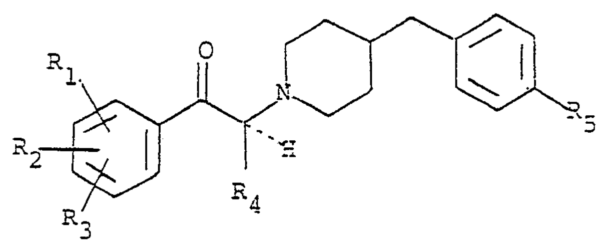

(IV)

gegebenenfalls nach Modifikation der Substituenten $R_1$, $R_2$ und $R_3$, einer Reduktion unterwirft und gewünschtenfalls die Substituenten $R_1$, $R_2$ und $R_3$ der so erhaltenen Verbindung der Formel I modifiziert.

2. Verfahren nach Anspruch 1, dadurch gekennzeichnet, daß man die Verbindungen der Formeln II und III in einem Lösungsmittel in Gegenwart einer schwachen Base reagieren läßt.

3. Verfahren nach Anspruch 1, dadurch gekennzeichnet, daß man die Verbindungen der Formeln II und III in Acetonitril in einem Verhältnis von 1 Mol des ersten zu 2 Molen des zweiten reagieren läßt.

4. Verfahren nach Anspruch 1, dadurch gekennzeichnet, daß man das Keton der Formel IV durch katalytische Hydrierung reduziert.

5. Verfahren nach Anspruch 1, dadurch gekennzeichnet, daß man das Keton der Formel IV mit Natrium- oder Kaliumborhydrid in saurem Medium reduziert.

6. Verfahren nach irgendeinem der Ansprüche 1 bis 5, dadurch gekennzeichnet, daß in Formel (I) $R_1$ ein Halogenatom bedeutet, $R_2$ und $R_3$ jeweils für ein Wasserstoffatom stehen, $R_4$ ein Wasserstoffatom oder eine Methylgruppe und $R_5$ ein Wasserstoff- oder ein Halogenatom bedeutet.